(19) Europäisches Patentamt
European Patent Office
Office européen des brevets

(11) EP 3 678 634 B1

(12) # EUROPÄISCHE PATENTSCHRIFT

(45) Veröffentlichungstag und Bekanntmachung des Hinweises auf die Patenterteilung:
**04.10.2023 Patentblatt 2023/40**

(21) Anmeldenummer: **18752494.7**

(22) Anmeldetag: **14.08.2018**

(51) Internationale Patentklassifikation (IPC):
***A61K 8/41*** *(2006.01)* ***A61K 8/34*** *(2006.01)*
***A61K 8/37*** *(2006.01)* ***A61K 8/45*** *(2006.01)*
***A61Q 5/02*** *(2006.01)* ***A61K 8/06*** *(2006.01)*

(52) Gemeinsame Patentklassifikation (CPC):
**A61K 8/416; A61K 8/068; A61K 8/345; A61K 8/37; A61K 8/45; A61Q 5/02;** A61K 2800/21

(86) Internationale Anmeldenummer:
**PCT/EP2018/071980**

(87) Internationale Veröffentlichungsnummer:
**WO 2019/048193 (14.03.2019 Gazette 2019/11)**

(54) **ZUSAMMENSETZUNG ENTHALTEND QUATERNÄRE AMMONIUMVERBINDUNG, INSBESONDERE ZUR HERSTELLUNG VON PFLEGE- UND REINIGUNGSFORMULIERUNGEN**

COMPOSITION CONTAINING QUATERNARY AMMONIUM COMPOUND, IN PARTICULAR FOR PREPARING PERSONAL CARE AND CLEANING FORMULATIONS

COMPOSITION CONTENANT UN COMPOSÉ D'AMMONIUM QUARTERNAIRE, EN PARTICULIER POUR LA PRODUCTION DE FORMULATIONS DE SOINS ET DE NETTOYAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.09.2017 EP 17189528**

(43) Veröffentlichungstag der Anmeldung:
**15.07.2020 Patentblatt 2020/29**

(73) Patentinhaber: **Evonik Operations GmbH**
**45128 Essen (DE)**

(72) Erfinder:
- **HARTUNG, Christian**
  **45133 Essen (DE)**
- **MENTEL, Matthias**
  **44357 Dortmund (DE)**
- **DAHL, Verena**
  **51429 Bergisch Gladbach (DE)**
- **SCHWAB, Peter**
  **45134 Essen (DE)**
- **KLOSTERMANN, Kristin**
  **45141 Essen (DE)**
- **WINTER, Patrick**
  **45473 Mülheim an der Ruhr (DE)**
- **KLEINEN, Jochen**
  **52525 Heinsberg (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 2 363 387** **DE-A1-102011 078 382**
**US-A1- 2013 012 423** **US-A1- 2015 297 485**

- **ANONYMOUS: "Kompatible Solute", IP.COM JOURNAL, IP.COM INC., WEST HENRIETTA, NY, US, 5. August 2002 (2002-08-05), XP013004047, ISSN: 1533-0001**
- **DATABASE GNPD [Online] MINTEL; 1. März 2006 (2006-03-01), "ColorVitality Blonde Leave-In Creme", XP002774691, Database accession no. 10253324**
- **DATABASE GNPD [Online] MINTEL; 1. Oktober 2008 (2008-10-01), "Glossing Reviving Serum", XP002774692, Database accession no. 994598**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 678 634 B1

## Beschreibung

### Gebiet der Erfindung

Gegenstand der Erfindung ist eine Zusammensetzung enthaltend

A) mindestens eine nicht silikonhaltige quaternäre Ammoniumverbindung,
B) mindestens ein kosmetisches Öl,
C) mindestens ein nicht-ionisches Tensid,
D) mindestens ein nicht-wässriges Lösungsmittel,
E) Wasser,

sowie die Verwendung solcher Zusammensetzungen, beispielsweise in einem Verfahren zur Herstellung von Formulierungen.

### Stand der Technik

Die WO2008155075 beschreibt eine kosmetische Zubereitung enthaltend (a) mindestens ein Tensid ausgewählt aus nicht-alkoxylierten anionischen, zwitterionischen oder amphoteren Tensiden, (b) eine Mikroemulsion und (c) mindestens ein kationisches Polymer.
Die EP1715833 beschreibt eine Mikroemulsion mit einem mittleren Teilchendurchmesser von 5 bis 250 nm enthaltend (a) 5 bis 50 Gew.% wenigstens eines bestimmten Alkyl- und/oder Alkenyloligoglykosidcarbonsäuresalzes, (b) 5 bis 50 Gew.% einer Ölkomponente und (c) 0 bis 15 Gew.% mono- und/oder polyfunktioneller Alkohole mit 1 bis 4 Kohlenstoffatomen, wobei die Summe der Komponenten (a) + (b) 10 bis 55 Gew.% der Gesamtzusammensetzung ausmacht.
Die WO2008155073 beschreibt eine kosmetische Zubereitung enthaltend (a) mindestens ein Tensid ausgewählt aus anionischen, zwitterionischen oder amphoteren Tensiden, (b) eine Mikroemulsion und (c) mindestens ein kationisches Polymer.
Die DE19755488 beschreibt Mikroemulsionen, enthaltend (a) 5 bis 30 Gew.-% Ölkörper, (b) 5 bis 80 Gew.-% anionische und/oder nicht-ionische Emulgatoren und (c) 12 bis 30 Gew.-% Polyole, mit der Maßgabe, dass sich die Mengenangaben mit Wasser zu 100 Gew.-% ergänzen.
US2013012423 offenbart die Verwendung von Mikroemulsionen, enthaltend (a) mindestens ein Alkyl(oligo)glycosid, (b) mindestens ein von (a) unterschiedliches Co-Tensid. (c) mindestens eine nicht-wasserlösliche organische Olkomponente, (d) mindestens ein Wachs und (e) Wasser, zur Herstellung von Reinigungsmitteln.
DE102011078382 offenbart Mikroemulsionen, welche als Öl-Phase ein mindestens eine quaternäre Ammoniumgruppe enthaltendes Polysiloxan aufweisen, Verfahren zu deren Herstellung sowie die Verwendung solcher Mikroemulsionen.
Nachteile der im Stand der Technik beschriebenen Zusammensetzungen sind, dass deren kosmetische Formulierungen eine nur geringe Konditionierwirkung auf Haut und Haaren aufweisen oder sie über eine nur geringe Affinität zu bestimmten Oberflächen verhältnismäßig verfügen.
Zudem erniedrigen die Zusammensetzungen des Standes der Technik die Viskosität in Tensid-Formulierungen wie Shampoos meist sehr stark und eine Verdickung der Formulierung ist extrem schwierig.
Aufgabe der Erfindung war es, mindestens einen der Nachteile des Standes der Technik zu überwinden.

### Beschreibung der Erfindung

Überraschenderweise wurde gefunden, dass die im Folgenden beschriebenen Zusammensetzungen die der Erfindung gestellte Aufgabe zu lösen vermögen.
Gegenstand der vorliegenden Erfindung sind Zusammensetzungen nach Anspruch 1 enthaltend mindestens eine nicht silikonhaltige quaternäre Ammoniumverbindung, mindestens ein kosmetisches Öl, mindestens ein nicht-ionisches Tensid, mindestens ein nicht-wässriges Lösungsmittel und Wasser.
Ein weiterer Gegenstand der Erfindung ist die Verwendung der erfindungsgemäßen Zusammensetzungen zur Herstellung von Formulierungen sowie die Formulierungen enthaltend die erfindungsgemäßen Mikroemulsionen.
Ein Vorteil der vorliegenden Erfindung ist es, dass sonst nicht wasserlösliche Öle in eine klare, wasserverdünnbare Form überführt werden können.
Ein weiterer Vorteil der vorliegenden Erfindung ist, dass Öle in kosmetischen Formulierungen eingearbeitet werden können, die in reiner Form oder in Lösungsmitteln verdünnt aufgrund der mangelnden Verträglichkeit nicht in kosmetische Formulierungen eingearbeitet werden können. Noch ein Vorteil der vorliegenden Erfindung ist, dass relativ

hohe Anteile an wasserunlöslichen Ölen klar und lagerstabil in kosmetische Formulierungen eingearbeitet werden können.

Ein weiterer Vorteil der vorliegenden Erfindung ist, dass die Mikroemulsionen aufgrund ihrer niedrigen Viskosität einfach zu verarbeiten und in Formulierungen einzuarbeiten sind. Es reicht einfaches Einrühren bei niedrigen Temperaturen, beispielsweise 25 °C.

Ein weiterer Vorteil der vorliegenden Erfindung ist, dass die wasserunlöslichen Öle in Formulierungen eine bessere Performance erreichen. Die Mikroemulsionen der vorliegenden Erfindung verstärken die Konditionierungseigenschaften der enthaltenen Öle wie Kämmbarkeit, Weichheit, Volumen, Formbarkeit, Handhabbarkeit, die Entwirrbarkeit von ungeschädigten und geschädigten Haaren und Glanzeffekt.

Noch ein Vorteil der vorliegenden Erfindung, dass die Formulierungen einen besonders guten konditionierenden Effekt in kosmetischen, dermatologischen und pharmazeutischen Formulierungen aufweisen.

Noch ein Vorteil der vorliegenden Erfindung ist, dass die Formulierungen ein angenehmes Hautgefühl in kosmetischen Formulierungen erzeugen können.

Ein weiterer Vorteil der vorliegenden Erfindung ist, dass die hier beschriebenen Formulierungen aus polyetherfreien Bestandteilen formuliert werden können und kosmetische Formulierungen bereitgestellt werden können, die im Wesentlichen frei von alkoxylierten Bestandteilen sind.

Ein weiterer Vorteil der erfindungsgemäßen Produkte ist, dass diese Silicon-frei sind, dabei aber ein sehr ähnliches sensorisches Profil auf dem Haar oder der Haut aufweisen.

Ein weiterer Vorteil der vorliegenden Erfindung ist, dass die hier beschriebenen Formulierungen den Glanz des Haares verbessern können.

Noch ein Vorteil der vorliegenden Erfindung ist, dass die Verdickbarkeit von den beschriebenen Formulierungen gut ist.

Ein weiterer Vorteil der vorliegenden Erfindung ist, dass weitere, frei wählbare sehr hydrophobe Öle wie Pflanzenöle zu einem gewissen Anteil klar eingearbeitet werden und damit ebenfalls leicht formuliert werden können.

Ein weiterer Vorteil der vorliegenden Erfindung ist, dass bei Einsatz in kosmetischen Formulierungen eine schnelle Trocknung von Haaren erreicht werden kann.

Noch ein Vorteil der vorliegenden Erfindung ist, dass die erfindungsgemäßen Zusammensetzungen ohne Konservierungsmittel auskommen.

Alle Bedingungen wie beispielsweise Druck und Temperatur sind, wenn nicht anders angegeben, Standardbedingungen (20 °C, 1 bar).

Im Zusammenhang mit der vorliegenden Erfindung sind unter dem Begriff "Fettsäuren" insbesondere Ameisensäure, Essigsäure, Propionsäure, Buttersäure, Valeriansäure, Capronsäure, Önanthsäure, Caprylsäure, Pelargonsäure, Caprinsäure, Laurinsäure, Myristinsäure, Pentadecansäure, Palmitinsäure, Margarinsäure, Stearinsäure, Nonadecansäure, Arachinsäure, Behensäure, Lignocerinsäure, Cerotinsäure, Montansäure, Melissinsäure, Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, Cervonsäure, Vernolsäure, Rizinolsäure zu verstehen, wobei solche mit einer Kettenlänge von 6 bis 24, bevorzugt 6 bis 22, insbesondere 8 bis 18, C-Atomen besonders bevorzugt sind; analoges gilt für das Kohlenstoffgrundgerüst für den im Zusammenhang mit der Erfindung verwendeten Begriff "Fettalkohole".

Alle angegebenen Prozent (%) sind, wenn nicht anders angegeben, Massenprozent.

Gegenstand der vorliegenden Erfindung ist eine Zusammensetzung enthaltend

A) mindestens eine nicht silikonhaltige quaternäre Ammoniumverbindung ausgewählt aus der Gruppe der Esterquats,
B) mindestens ein kosmetisches Öl umfassend keine Silikonöle,
C) mindestens ein nicht-ionisches Tensid,
D) mindestens ein nicht-wässriges Lösungsmittel,
E) Wasser, dadurch gekennzeichnet, dass sie eine Mikroemulsion ist.

Mikroemulsionen sind thermodynamisch stabile Mischungen aus Wasser (wässrige Phase), Öl (nicht mit Wasser mischbare Phase) und Tensid (Solubilisator).

Erfindungsgemäß bevorzugte Mikroemulsionen weisen eine Domänengröße der dispersen Phase von kleiner 1000 nm, insbesondere kleiner 500 nm auf, wobei die Bestimmung der Domänengröße mit Hilfe dem Fachmann bekannter Streumethoden durchgeführt wird, wie beispielsweise in P. Lindner und Th. Zemb, "Neutrons, X-Rays and Light: Scattering Methods Applied to Soft Condensed Matter", Elsevier Science & Technology, November 2002 oder O. Glatter und O. Kratky, "Small-angle X-ray Scattering" Academic Press Inc, Dezember 1982, beschrieben.

Insbesondere für kosmetische oder topische Anwendungen sind Zusammensetzungen bevorzugt, die im Wesentlichen frei von alkoxylierten Verbindungen sind. Unter dem Begriff "im Wesentlichen frei von alkoxylierten Verbindungen" im Zusammenhang mit der vorliegenden Erfindung ist zu verstehen, dass die Zusammensetzung keine nennenswerten Mengen an alkoxylierten Verbindungen aufweist, die eine oberflächenaktive Wirkung ausüben. Insbesondere ist hierunter zu verstehen, dass alkoxylierte Verbindungen in Mengen von kleiner 1 Gew.-%, bevorzugt von kleiner 0,1 Gew.-%, besonders bevorzugt von kleiner 0,01 Gew.-% bezogen auf die Gesamtzusammensetzung, insbesondere keine nachweisbaren Mengen enthalten sind.

Eine bevorzugte Zusammensetzung der vorliegenden Erfindung ist dadurch gekennzeichnet, dass

A) in einer Menge von 1 Gew.-% bis 40 Gew.-%, bevorzugt in einer Menge von 1,5 Gew.-% bis 30 Gew.-%, besonders bevorzugt in einer Menge von 5 Gew.-% bis 25 Gew.-%,
B) in einer Menge von 5 Gew.-% bis 60 Gew.-%, bevorzugt in einer Menge von 10 Gew.-% bis 50 Gew.-%, besonders bevorzugt in einer Menge von 15 Gew.-% bis 40 Gew.-%,
C) in einer Menge von 5 Gew.-% bis 50 Gew.-%, bevorzugt in einer Menge von 10 Gew.-% bis 40 Gew.-%, besonders bevorzugt in einer Menge von 15 Gew.-% bis 35 Gew.-%,
D) in einer Menge von 2 Gew.-% bis 50 Gew.-%, bevorzugt in einer Menge von 3 Gew.-% bis 40 Gew.-%, besonders bevorzugt in einer Menge von 5 Gew.-% bis 30 Gew.-%, und
E) in einer Menge von 1 Gew.-% bis 60 Gew.-%, bevorzugt in einer Menge von 3 Gew.-% bis 50 Gew.-%, besonders bevorzugt in einer Menge von 5 Gew.-% bis 45 Gew.-%,

enthalten ist, wobei sich die Gew.-% auf die Gesamtzusammensetzung beziehen und insbesondere mit der Maßgabe, dass die Masse der Komponente B) größer als die Masse der Komponente A) in der Zusammensetzung ist.

Eine bevorzugte Zusammensetzung der vorliegenden Erfindung ist dadurch gekennzeichnet, dass

A) ausgewählt ist aus der Gruppe der Unter dem Begriff "Esterquat" im Zusammenhang mit der vorliegenden Erfindung versteht man eine chemische Verbindung, die sowohl ein quartäres Stickstoffatom als auch eine Esterbindung im kationischen Teil eines Ionenpaares enthält. Bevorzugt versteht man darunter eine Klasse von oberflächenaktiven quaternären Ammoniumverbindungen mit der allgemeinen Formel $R^{11}R^{12}R^{13}R^{14}N^+ X^-$, dadurch gekennzeichnet, dass mindestens einer der Reste $R^{11}$ bis $R^{14}$, der dadurch gekennzeichnet ist, dass er mehr als 4 C-Atome aufweist, an die geladene Gruppe über Esterbindungen C (O) O- oder OC (O) - gebunden ist, und unter X-jedes anionisches Gegenion verstanden wird.

$R^{11}$ ein gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest
$R^{12}$ ein gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest
$R^{13}$ ein gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest
$R^{14}$ ein gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest.

Unter dem Begriff "flüssige Esterquats" im Zusammenhang mit der vorliegenden Erfindung werden Esterquats, die bei 1 bar einen Schmelzpunkt von 40 °C und kleiner, besonders bevorzugt 25 °C oder kleiner und ganz besonders bevorzugt 10 °C oder kleiner verstanden. Handelt es sich bei den in der Zusammensetzung enthaltenen Esterquats um Mischungen von Esterquats, so bezieht sich der Schmelzpunkt auf den Schmelzpunkt der Mischung aller in der Formulierung enthaltenen Esterquats. Analoges gilt für Imidazoliniumsalze.

Eine bevorzugte Zusammensetzung der vorliegenden Erfindung ist dadurch gekennzeichnet, dass

A) ausgewählt ist aus der Gruppe der flüssigen Esterquats bestehend aus quaternierten Fettsäurealkanolaminester-Salze, besonders bevorzugt aus den Gruppen der quaternierten Fettsäureethanolaminester-Salze und der quaternierten Fettsäureisopropanolaminester-Salze, ganz besonders bevorzugt aus der Gruppe der quaternierten Fettsäureisopropanolaminester-Salze auf Basis von Di-Methyl-Mono-, Methyl-Di- oder Tri-isopropanol-Amin.

Dies hat den vorteilhaften Effekt einer verbesserten Langzeitlagerstabilität der erfindungsgemäßen Zusammensetzung.

Eine insbesondere bevorzugte Zusammensetzung der vorliegenden Erfindung ist dadurch gekennzeichnet, dass

A) ausgewählt ist aus der Gruppe der flüssigen Esterquats umfassend Verbindungen der allgemeinen Formel (I)

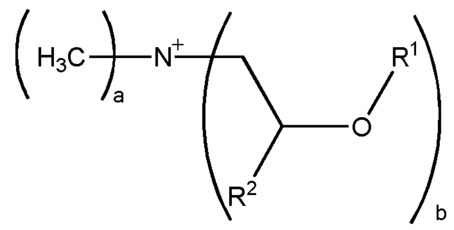

$X^-$ allgemeine Formel (I)

mit $R^1$ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit $R^2$ ein Alkylrest mit 1 bis 6 Kohlenstoffatomen, bevorzugt Methyl, Ethyl, Propyl, Isopropyl, besonders bevorzugt Methyl, und
mit $X^-$ ein Gegenion zu der positiven Ladung an der quartären Stickstoffgruppe, wobei hier auch zweifach oder dreifach negativ geladene Ionen einbezogen sein sollen,
wobei a = 1 bis 3 und b = 1 bis 3, bevorzugt a = 1,7 bis 2,3 und b = 1,7 bis 2,3
mit der Maßgabe, dass a + b =4.

Die erfindungsgemäße Zusammensetzung ist nach außen bezüglich ihrer elektrischen Ladung neutral, da die Ladungen der Esterquats durch entsprechende Gegenionen X- neutralisiert werden. Erfindungsgemäß geeignet sind alle Gegenionen, die die Ladung der Quats kompensieren können. Bevorzugt ist $X^-$ im Zusammenhang mit der vorliegenden Erfindung ausgewählt aus der Gruppe der kosmetisch akzeptablen Anionen, insbesondere der Gruppe Chlorid, Sulfat, Phosphat Methylsulfat, Ethylsulfat, Methansulfonat, Ethansulfonat, Tosylat, Acetat, Lactat und Citrat.

Für den Fall, dass b>1 ist, können die Reste $R^1$ gleich oder ungleich sein.

$R^1$ als Acylrest einer mindestens einfach ungesättigten Fettsäure mit einer Kettenlänge von 18 bis 24 Kohlenstoffatomen kann eine oder mehr, beispielsweise zwei oder drei Doppelbindungen enthalten.

Erfindungsgemäß bevorzugte Zusammensetzungen sind dadurch gekennzeichnet, dass $R^1$ als Acylrest einer mindestens einfach ungesättigten Fettsäure mit einer Kettenlänge von 18 bis 24 Kohlenstoffatomen ausgewählt ist aus den Acylresten der Säuren aus der Gruppe umfassend Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, und Cervonsäure, wobei Ölsäure besonders bevorzugt ist.

Es können erfindungsgemäß auch Mischungen dieser Carbonsäuren eingesetzt werden. Bevorzugte erfindungsgemäße Zusammensetzungen enthalten mindestens eine Verbindung der allgemeinen Formel (I) mit a = 1,7 bis 2,3 und b = 1,7 bis 2,3, besonders bevorzugt a = b = 2. Eine erfindungsgemäß besonders bevorzugte Zusammensetzung ist dadurch gekennzeichnet, dass $R^1$ der Acylrest der Ölsäure und a = 1,7 bis 2,3 und b = 1,7 bis 2,3, besonders bevorzugt a = b = 2, ist.

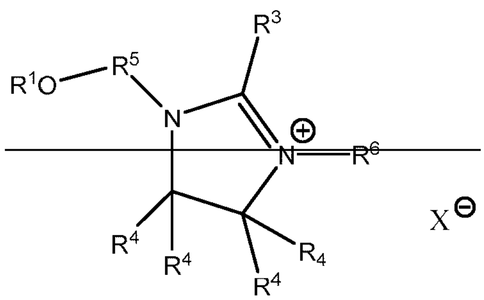

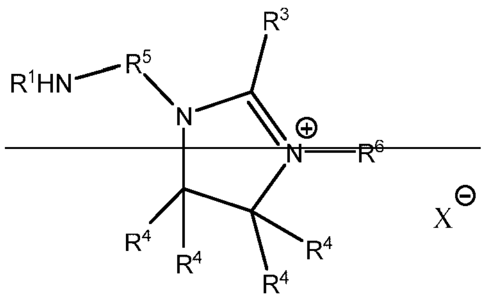

Komponente B) ist ein kosmetisches Öl.

Unter dem Begriff "kosmetisches Öl" im Zusammenhang mit der vorliegenden Erfindung sind mit Wasser nichtmischbare Flüssigkeiten, welche zur Herstellung kosmetischer Formulierungen geeignet sind, zu verstehen. Mit Wasser nicht mischbar bedeutet im Zusammenhang mit der vorliegenden Erfindung, dass bei Raumtemperatur wässrige Mischungen von kosmetischen Ölen bei Ölkonzentrationen von 0,5 - 99,5 vol.-% bezogen auf die Gesamtmischung zu einer bereits mit dem menschlichen Auge wahrnehmbaren Trübung bzw. zur Ausbildung von zwei oder mehr Phasen führen. Des Weiteren sind im Zusammenhang mit der vorliegenden Erfindung kosmetische Öle bevorzugt dadurch gekennzeichnet, dass sie zu Wasser ein Grenzflächenspannung von > 5 mN/m aufweisen. Kosmetische Öle können zum Beispiel Oleochemie- oder Silikonchemie basiert sein.

Erfindungsgemäß bevorzugt sind in der erfindungsgemäßen Zusammensetzung kosmetische Öle enthalten ausgewählt aus der Gruppe der Fettalkohole, Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen, Ester von Alkylhydroxycarbonsäuren mit linearen oder verzweigten Fettalkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen. Des Weiteren Ether, mit oder ohne Alkoxygruppen, Des Weiteren Mischungen dieser Öle in beliebigen Verhältnissen. Bevorzugt Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen, besonders bevorzugt lineare, cyclische oder verzweigte Kohlenwasserstoffe, mit oder ohne Substituenten, mit oder ohne Doppelbindungen. Des Weiteren Carbonate mit unverzweigten oder verzweigten Alkoholen, Carbonate mit unverzweigten oder verzweigten mehrwertigen Alkoholen, Carbonate mit linearen oder verzweigten Fettalkoholen. Ester von verzweigten Fettsäuren mit unverzweigten oder verzweigten mehrwertigen Alkoholen.

Es ist erfindungsgemäß wenn Komponente B), das kosmetisches Öl, keine Silikonöle umfasst.

Insbesondere ist es auch vorteilhaft und daher bevorzugt, wenn die erfindungsgemäßen Zusammensetzungen gar keine silikonhaltigen Komponenten umfassen. Diese sind somit silikonfrei.

Im Zusammenhang mit den silikonfreien erfindungsgemäßen Zusammensetzungen ist es erfindungsgemäß bevorzugt, wenn die Komponente A) ausgewählt ist aus Esterquats, wobei oben als bevorzugt enthalten beschriebene Esterquats auch im Zusammenhang mit den silikonfreien erfindungsgemäßen Zusammensetzungen entsprechend bevorzugt enthalten sind.

Komponente C) ist erfindungsgemäß mindestens ein nicht-ionisches Tensid.

Bevorzugte nicht-ionische Tenside sind ausgewählt aus der Gruppe enthaltend, bevorzugt bestehend aus,

Anlagerungsprodukten von Ethylenoxid und/oder Propylenoxid an lineare Fettalkohole, Fettsäuren, Fettsäureamide,

Fettamine und an Alkylphenole,
Glycerinmono- und -diester und Sorbitanmono- und -diester von gesättigten und ungesättigten Fettsäuren und deren Ethylenoxidanlagerungsprodukte,
Alkylmono- und -oligoglycoside und deren Ethylenoxidanlagerungsprodukte, Anlagerungsprodukte von Ethylenoxid an Rizinusöl und/oder gehärtetes Rizinusöl,
Partialester auf Basis linearer, verzweigter, ungesättigter bzw. gesättigter Fettsäuren, Ricinolsäure, 12-Hydroxystearinsäure, Glycerin, Polyglycerin, Pentaerythrit, Dipentaerythrit und Zuckeralkohole (z.B. Sorbit), Alkylglucoside (z.B. Methylglucosid, Butylglucosid, Laurylglucosid) sowie Polyglucoside (z.B. Cellulose), Mono-, Di- und Trialkylphosphate sowie Mono-, Di- und/oder Tri-PEG-alkylphosphate und deren Salze, Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate,
weiter alkoxylierte Triglyceride, Mischether bzw. Mischformale, gegebenenfalls partiell oxidierte Alkyloligoglykoside oder Alkenyloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Polysorbate und Aminoxide und Mischungen dieser Tenside.

Sofern die nicht-ionischen Tenside Polyglycoletherketten enthalten, können diese eine konventionelle, vorzugsweise jedoch eine eingeengte Homologenverteilung aufweisen.

Besonders bevorzugte nicht-ionische Tenside als Komponente C) sind Polyglycerinester. Unter dem Begriff "Polyglycerinester" im Zusammenhang mit der vorliegenden Erfindung werden Polyglycerinpartialester, somit Verbindungen bei denen nicht alle Hydroxylgruppen verestert wurden, mitumfasst.

Erfindungsgemäß bevorzugt ist der Polyglycerinester der Komponente C) ausgewählt aus solchen der allgemeinen Formel IV

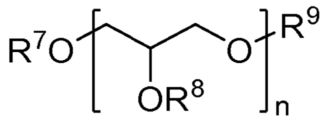

allgemeine Formel IV
mit

n = 2 bis 16, bevorzugt 3 - 14, besonders bevorzugt 4 - 11, und
$R^7$, $R^8$, $R^9$ = unabhängig voneinander gleich oder verschieden ausgewählt aus H und gegebenenfalls mindestens eine Hydroxylgruppe enthaltender, gesättigter oder ungesättigter, linearer oder verzweigter Acylrest mit 4 - 36 C-Atomen, insbesondere ausgewählt aus den Acylresten natürlicher Fettsäuren.

Erfindungsgemäß bevorzugt eingesetzte Polyglycerinester der allgemeinen Formel IV sind dadurch gekennzeichnet, dass das Gewichtsverhältnis des Polyglycerylrests zur Summe der Acylreste $R^7$; $R^8$ und $R^9$ 90:10 bis 45:55, bevorzugt 88:12 bis 50:50 beträgt.

$R^7$ ist bevorzugt H und die Reste $R^8$ und $R^9$ sind bevorzugt H oder Acylreste von natürlichen Fettsäuren. $R^8$ und $R^9$ können auch Mischungen solcher Acylreste repräsentieren, insbesondere technische Mischungen wie beispielsweise Kokosfettsäureschnitte.

Für $R^8$ und $R^9$ ist es insbesondere bevorzugt, dass bezogen auf alle Reste $R^8$ und $R^9$ im Polyglycerinester mindestens 50 Mol-%, bevorzugt mindestens 75 Mol-% der Acylreste $R^9$ ausgewählt sind aus Capryloyl-, Caproyl- und Lauroylresten.

Dem Fachmann ist bewusst, dass das in der allgemeinen Formel IV enthaltene Polyglycerin-Grundgerüst aufgrund seiner polymeren Eigenschaft eine statistische Mischung verschiedener Verbindungen darstellt. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. "Original synthesis of linear, branched and cyclic oligoglycerol standards", Cassel et al., J. Org. Chem. 2001, 875-896.

Entsprechende Strukturen sind von der in dieser Hinsicht vereinfachten, allgemeinen Formel IV miterfasst.

Der Polymerisationsgrad n lässt sich dadurch bestimmen, dass die Hydroxylzahl des zur Synthese des erfindungsgemäßen Esters eingesetzten Polyglycerins ermittelt wird, wobei der mittlere Polymerisationsgrad n mit der Hydroxylzahl des zugrundeliegenden Polyglycerins über folgende Gleichung verknüpft ist:

$$n = \frac{\frac{2000 \bullet M(KOH)}{OHZ} - M(Wasser)}{\left[ [M(Glycerin) - M(Wasser)] - \frac{1000 \bullet M(KOH)}{OHZ} \right]}$$

mit M = molare Masse; OHZ = Hydroxylzahl des freien Polyglycerins.

Alternativ lässt sich der Polymerisationsgrad n auch dadurch bestimmen, dass die Hydroxylzahl des nach vollständiger Esterhydrolyse erhaltenen Polyglycerins ermittelt wird.

Geeignete Bestimmungsmethoden zur Ermittlung der Hydroxylzahl sind insbesondere solche gemäß DGF C-V 17 a (53), Ph. Eur. 2.5.3 Method A und DIN 53240.

Eine bevorzugte Zusammensetzung der vorliegenden Erfindung ist dadurch gekennzeichnet, dass
D) ein nicht-wässriges Lösungsmittel enthalten ist, ausgewählt aus der Gruppe enthaltend, bevorzugt bestehend aus,

Hydrotropen beispielsweise aus der Gruppe der aliphatischen Alkohole, wie Ethanol, Propanol oder 1,3-Propandiol, zyklische Carbonate wie Ethylencarbonat, Propylencarbonat, Glycerincarbonat, Ester von Mono- oder Polycarbonsäuren wie Ethylacetat, Ethyllactat, Glycerin, Isopropylalkohol, Dipropylenglykol, Glykolether (beispielsweise unter dem Namen DOWANOL® von Dow Chemicals erhältlich) und Polyole. Polyole, die hier in Betracht kommen, können 2 bis 15 Kohlenstoffatome und mindestens zwei Hydroxylgruppen besitzen. Typische Beispiele sind: Glycerin, Alkylenglycole, wie beispielsweise Ethylenglycol, Diethylenglycol, Propylenglycol, Butylenglycol, Pentylenglycol, Hexylenglycol, 1,2-Propandiol, 1,2-Butandiol, 1,2-Pentandiol, 1,2-Hexandiol sowie Polyethylenglycol oder Polypropylenglycol, Polyhydroxycarbonsäuren, Butyldiglykol und Gemische dieser Lösungsmittel.

Insbesondere bevorzugt ist das nicht-wässrige Lösungsmittel D) ausgewählt aus der Gruppe bestehend aus Glycerin, Glykol, 1,2-Propylenglykol, 1,3-Propylenglykol, Butylenglycol oder Dipropylenglykol.

Eine weitere bevorzugte Zusammensetzung der vorliegenden Erfindung ist dadurch gekennzeichnet, dass sie als zusätzliche Komponente F) ein amphoteres Tensid, insbesondere in bis zu 10% bezogen auf die Gesamtzusammensetzung, enthält. Typische Beispiele für amphotere Tenside sind Amphoacetate, Amphopropionate, Alkylbetaine, Alkylamidobetaine, Aminopropionate, Aminoglycinate, Imidazoliniumbetaine und Sulfobetaine, wie z.B. die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein insbesondere bevorzugtes amphoteres Tensid ist das unter der INCI-Bezeichnung bekannte Cocamidopropyl Betaine.

Die Zusammensetzungen der vorliegenden Erfindung, insbesondere die erfindungsgemäßen Mikroemulsionen, lassen sich vorteilhaft zur Herstellung von Pflege- und Reinigungsformulierungen, insbesondere für Haut und Hautanhangsgebilden, wie beispielsweise Konditioniermittel für Haare, verwenden, wobei diese bevorzugt ausgewählt sind aus der Gruppe der kosmetischen, reinigenden und pflegenden Formulierungen. Somit sind derartige Verwendungen ebenfalls Gegenstand der vorliegenden Erfindung.

Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt. In diesem Zusammenhang können die Pflege- und Reinigungsformulierungen kosmetische, pharmazeutische oder dermatologische Formulierungen wie z. B. zur Behandlung der Haare in Form von Haarshampoos, 2in1-Shampoos, Flüssigseifen, Haarspülungen, Dauerwell-Fixierlösungen, Haarfärbeshampoos, Haarfestiger, Haarkuren, Haarlegemittel, Haarstyling-Zubereitungen, Fön-Lotionen, Schaumfestiger, Leave-In Conditionierer, Haarglättungsmitteln, Glanzverbesserungsmitteln und Mittel zum Färben der Haare darstellen.

Ein weiterer Gegenstand dieser Erfindung ist die Verwendung der erfindungsgemäßen Mikroemulsionen als Konditioniermittel für Haarbehandlungsmittel und Haarnachbehandlungsmittel sowie als Mittel zur Verbesserung der Haarstruktur.

Die erfindungsgemäße Verwendung kann beispielsweise in Form eines erfindungsgemäßen Verfahrens zur Herstellung von Pflege- und Reinigungsformulierungen stattfinden, welches gekennzeichnet ist durch die Verfahrensschritte:

1) Bereitstellen einer erfindungsgemäßen Zusammensetzung,

2) Vermischen mit einer wasserhaltigen Phase, bevorzugt umfassend mindestens ein Konservierungsmittel und/oder mindestens ein Parfüm.

Unter dem Begriff "Konservierungsmittel" im Zusammenhang mit der vorliegenden Erfindung ist ein Mittel zu verstehen, welches hinsichtlich mikrobiellem, insbesondere bakteriellem, Bewuchs konserviert.

Noch ein Gegenstand der vorliegenden Erfindung sind somit auch die Pflege- und Reinigungsformulierungen, insbesondere für Haut und Hautanhangsgebilden, insbesondere kosmetische Formulierungen, wobei diese bevorzugt ausgewählt sind aus der Gruppe der Haarbehandlungsmittel und Haarnachbehandlungsmittel zum Ausspülen oder zum Verbleib im Haar, beispielsweise Shampoos mit oder ohne ausgeprägter Konditionierwirkung, 2in1-Shampoos, Spülungen, Haarkuren, Haarmasken, Frisierhilfen, Stylingmittel, Fönlotionen, Haarfestiger, Dauerwellmittel, Haarglättungsmittel und Mittel zum Färben der Haare, insbesondere Konditioniermittel und Shampoos enthaltend mindestens eine erfindungsgemäße Zusammensetzungen.

Erfindungsgemäß besonders bevorzugte kosmetische Formulierungen sind selber auch im Wesentlichen frei von alkoxylierten Verbindungen.

Eine erfindungemäß bevorzugte Formulierung enthält die erfindungsgemäße Mikroemulsion in einer Menge von 0,1 Gew.-% bis 99 Gew.-%, bevorzugt in einer Menge von 0,5 Gew.-% bis 20 Gew.-%, besonders bevorzugt in einer Menge von 1,0 Gew.-% bis 10 Gew.-%, wobei sich die Gew.-% auf die Gesamtformulierung beziehen.

Die erfindungsgemäße Formulierung, insbesondere die kosmetisch Formulierung, kann zum Beispiel mindestens eine zusätzliche Komponente enthalten, ausgewählt aus der Gruppe der Emollients,

Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung. Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

Abbildung 1: Wassergehalt der Haarsträhnen nach Anwendung von 1 g einer verdünnten Formulierung

Beispiele:

*Beispiel ME1:* Nicht erfindungsgemäße *Mikroemulsion:*

25,0% TEGOSOFT M (Isopropyl Myristate, Evonik Nutrition & Care GmbH), 12,5% VARISOFT PATC (Palmitamidopropyltrimonium Chloride, Evonik Nutrition & Care GmbH), 10,0% Dipropylenglykol und 30,0% TEGOSOFT PC 31 (Polyglyceryl-3 Caprate, Evonik Nutrition & Care GmbH) wurden in 22,5% Wasser verrührt. Es bildet sich eine klare Mikroemulsion aus. Der Anteil an signifikant haarkonditionieraktiven Substanzen (TEGOSOFT M und VARISOFT PATC) beträgt 37,5%.

*Beispiel ME2: Erfindungsgemäße Mikroemulsion:*

26,7% TEGOSOFT M (Isopropyl Myristate, Evonik Nutrition & Care GmbH), 13,3% VARISOFT EQ 100 (Bis-(Isostearoyl/Oleoyl Isopropyl) Dimonium Methosulfate, Evonik Nutrition & Care GmbH), 24,0% Dipropylenglykol und 26,7% TEGOSOFT PC 41 (Polyglyceryl-4 Caprate, Evonik Nutrition & Care GmbH) wurden in 9,3% Wasser verrührt. Es bildet sich eine klare Mikroemulsion aus. Der Anteil an signifikant haarkonditionieraktiven Substanzen (TEGOSOFT M und VARISOFT EQ 100) beträgt 40%.

*Beispiel ME3:* Nicht erfindungsgemäße *Mikroemulsion:*

25,0% TEGOSOFT M (Isopropyl Myristate, Evonik Nutrition & Care GmbH), 2,5% VARISOFT PATC (Palmitamidopropyltrimonium Chloride, Evonik Nutrition & Care GmbH), 10,0% Propylenglykol, 20,0% TEGOSOFT PC 31 (Polyglyceryl-3 Caprate, Evonik Nutrition & Care GmbH) und 15,0% TEGOSOFT PC 41 (Polyglyceryl-4 Caprate, Evonik Nutrition & Care GmbH) wurden in 27,5% Wasser verrührt. Es bildet sich eine klare Mikroemulsion aus. Der Anteil an signifikant haarkonditionieraktiven Substanzen (TEGOSOFT M und VARISOFT PATC) beträgt 27,5%.

*Beispiel ME4: Nicht erfindungsgemäße Mikroemulsion:*

Plantasil Micro (Dicaprylyl Ether (and) Decyl Glucoside (and) Glyceryl Oleate, BASF), mit ca. 28% haarkonditionieraktiven Bestandteilen (Dicaprylyl Ether und Glyceryl Oleate).

*Beispiel ME5: Nicht erfindungsgemäße Mikroemulsion:*

Lamesoft OD (Coco-Caprylate (and) Lauryl Glucoside (and) Glycerin (and) Polyglyceryl-2 Dipolyhydroxystearate (and) Polyglyceryl-3 Diisostearate, BASF).

*Beispiel ME6: Erfindungsgemäße Mikroemulsion:*

23,9% TEGOSOFT CT (Caprylic/Capric Triglyceride, Evonik Nutrition & Care GmbH), 21,0% TEGOSOFT PC 31 (Polyglyceryl-3 Caprate, Evonik Nutrition & Care GmbH), 9,0% VARISOFT EQ 100 (Bis-(Isostearoyl/Oleoyl Isopropyl) Dimonium Methosulfate, Evonik Nutrition & Care GmbH) und 18,0% Dipropylenglykol wurden in 28,1% Wasser verrührt. Es bildet sich eine klare Mikroemulsion aus. Der Anteil an signifikant haarkonditionieraktiven Substanzen (TEGOSOFT CT und VARISOFT EQ 100) beträgt 32,9%.

*Beispiel ME7: Erfindungsgemäße Mikroemulsion:*

21,0% TEGOSOFT DEC (Diethylhexyl Carbonate, Evonik Nutrition & Care GmbH), 18,0% TEGOSOFT PC 31 (Polyglyceryl-3 Caprate, Evonik Nutrition & Care GmbH), 8,0% VARISOFT EQ 100 (Bis-(Isostearoyl/Oleoyl Isopropyl) Dimonium Methosulfate, Evonik Nutrition & Care GmbH) und 15,6% Dipropylenglykol wurden in 37,4% Wasser verrührt. Es bildet sich eine klare Mikroemulsion aus. Der Anteil an signifikant haarkonditionieraktiven Substanzen (TEGOSOFT DEC und VARISOFT EQ 100) beträgt 29,0%.

*Beispiel ME8: Erfindungsgemäße Mikroemulsion:*

19,8% TEGOSOFT DEC (Diethylhexyl Carbonate, Evonik Nutrition & Care GmbH), 17,3% TEGOSOFT PC 31 (Polyglyceryl-3 Caprate, Evonik Nutrition & Care GmbH), 5,0% TEGO Betain F 50 (Cocamidopropyl Betaine, Evonik

Nutrition & Care GmbH), 7,0% VARISOFT EQ 100 (Bis-(Isostearoyl/Oleoyl Isopropyl) Dimonium Methosulfate, Evonik Nutrition & Care GmbH) und 14,0% Dipropylenglykol wurden in 36,9% Wasser verrührt. Es bildet sich eine klare Mikroemulsion aus. Der Anteil an signifikant haarkonditionieraktiven Substanzen (TEGOSOFT DEC und VARISOFT EQ 100) beträgt 26,8%.

*Beispiel ME9: Erfindungsgemäße Mikroemulsion:*

15,8% TEGOSOFT OER (Oleyl Erucate, Evonik Nutrition & Care GmbH), 15,8% TEGOSOFT M (Isopropyl Myristate, Evonik Nutrition & Care GmbH), 22,8% TEGO Solve 61 (Polyglyceryl-6 Caprylate (and) Polyglyceryl-3 Cocoate (and) Polyglyceryl-4 Caprate (and) Polyglyceryl-6 Ricinoleate, Evonik Nutrition & Care GmbH), 22,8% VARISOFT EQ 100 (Bis-(Isostearoyl/Oleoyl Isopropyl) Dimonium Methosulfate, Evonik Nutrition & Care GmbH) und 10,1% Dipropylenglykol wurden in 12,7% Wasser verrührt. Es bildet sich eine klare Mikroemulsion aus. Der Anteil an signifikant haarkonditionieraktiven Substanzen (TEGOSOFT OER, TEGOSOFT M und VARISOFT EQ 100) beträgt 54,4%.

*Beispiel ME10: Erfindungsgemäße Mikroemulsion:*

15,8% TEGOSOFT OER (Oleyl Erucate, Evonik Nutrition & Care GmbH), 15,8% TEGOSOFT M (Isopropyl Myristate, Evonik Nutrition & Care GmbH), 22,8% TEGOSOFT PC 41 (Polyglyceryl-4 Caprate, Evonik Nutrition & Care GmbH), 22,8% VARISOFT EQ 100 (Bis-(Isostearoyl/Oleoyl Isopropyl) Dimonium Methosulfate, Evonik Nutrition & Care GmbH) und 10,1% Dipropylenglykol wurden in 12,7% Wasser verrührt. Es bildet sich eine klare Mikroemulsion aus. Der Anteil an signifikant haarkonditionieraktiven Substanzen (TEGOSOFT OER, TEGOSOFT M und VARISOFT EQ 100) beträgt 54,4%.

*Beispiel ME11: Erfindungsgemäße Mikroemulsion:*

25,0% TEGOSOFT P (Isopropyl Palmitate, Evonik Nutrition & Care GmbH), 18,0% TEGOSOFT PC 41 (Polyglyceryl-4 Caprate, Evonik Nutrition & Care GmbH), 18,0% VARISOFT EQ 100 (Bis-(Isostearoyl/Oleoyl Isopropyl) Dimonium Methosulfate, Evonik Nutrition & Care GmbH) und 8,0% Dipropylenglykol wurden in 31,0% Wasser verrührt. Es bildet sich eine klare Mikroemulsion aus. Der Anteil an signifikant haarkonditionieraktiven Substanzen (TEGOSOFT P und VARISOFT EQ 100) beträgt 43%.

*Beispiel ME12: Erfindungsgemäße Mikroemulsion:*

8,3% TEGOSOFT OER (Oleyl Erucate, Evonik Nutrition & Care GmbH), 22,9% TEGOSOFT P (Isopropyl Palmitate, Evonik Nutrition & Care GmbH), 16,5% TEGOSOFT PC 41 (Polyglyceryl-4 Caprate, Evonik Nutrition & Care GmbH), 16,5% VARISOFT EQ 100 (Bis-(Isostearoyl/Oleoyl Isopropyl) Dimonium Methosulfate, Evonik Nutrition & Care GmbH) und 7,3% Dipropylenglykol wurden in 28,5% Wasser verrührt. Es bildet sich eine klare Mikroemulsion aus. Der Anteil an signifikant haarkonditionieraktiven Substanzen (TEGOSOFT OER, TEGOSOFT P und VARISOFT EQ 100) beträgt 47,7%.

*Beispiel ME13: Erfindungsgemäße Mikroemulsion:*

6,3% TEGOSOFT OER (Oleyl Erucate, Evonik Nutrition & Care GmbH), 18,7% TEGOSOFT P (Isopropyl Palmitate, Evonik Nutrition & Care GmbH), 18,0% TEGO Solve 61 (Polyglyceryl-6 Caprylate (and) Polyglyceryl-3 Cocoate (and) Polyglyceryl-4 Caprate (and) Polyglyceryl-6 Ricinoleate, Evonik Nutrition & Care GmbH), 18,0% VARISOFT EQ 100 (Bis-(Isostearoyl/Oleoyl Isopropyl) Dimonium Methosulfate, Evonik Nutrition & Care GmbH) und 8,0% Dipropylenglykol wurden in 31,0% Wasser verrührt. Es bildet sich eine klare Mikroemulsion aus. Der Anteil an signifikant haarkonditionieraktiven Substanzen (TEGOSOFT OER, TEGOSOFT P und VARISOFT EQ 100) beträgt 43%.

*Beispiel ME14: Erfindungsgemäße Mikroemulsion:*

15,8% TEGOSOFT OER (Oleyl Erucate, Evonik Nutrition & Care GmbH), 15,8% TEGOSOFT P (Isopropyl Palmitate, Evonik Nutrition & Care GmbH), 11,4% TEGO Solve 61 (Polyglyceryl-6 Caprylate (and) Polyglyceryl-3 Cocoate (and) Polyglyceryl-4 Caprate (and) Polyglyceryl-6 Ricinoleate, Evonik Nutrition & Care GmbH), 11,4% TEGOSOFT PC 41 (Polyglyceryl-4 Caprate, Evonik Nutrition & Care GmbH), 22,8% VARISOFT EQ 100 (Bis-(Isostearoyl/Oleoyl Isopropyl) Dimonium Methosulfate, Evonik Nutrition & Care GmbH) und 10,1% Dipropylenglykol wurden in 12,7% Wasser verrührt. Es bildet sich eine klare Mikroemulsion aus. Der Anteil an signifikant haarkonditionieraktiven Substanzen (TEGOSOFT OER, TEGOSOFT P und VARISOFT EQ 100) beträgt 54,4%.

*Beispiel ME15:* Nicht erfindungsgemäße *Mikroemulsion:*

25,0% TEGOSOFT DEC (Diethylhexyl Carbonate, Evonik Nutrition & Care GmbH), 30,0% TEGOSOFT PC 31 (Polyglyceryl-3 Caprate, Evonik Nutrition & Care GmbH), 5,0% VARISOFT PATC (Palmitamidopropyltrimonium Chloride, Evonik Nutrition & Care GmbH) und 15,0% Propylenglykol wurden in 25,0% Wasser verrührt. Es bildet sich eine klare Mikroemulsion aus. Der Anteil an signifikant haarkonditionieraktiven Substanzen (TEGOSOFT DEC und VARISOFT PATC) beträgt 30,0%.

*Beispiel ME16: Erfindungsgemäße Mikroemulsion:*

18,4% TEGOSOFT AC (Isoamyl Cocoate, Evonik Nutrition & Care GmbH), 20,1% TEGOSOFT PC 41 (Polyglyceryl-4 Caprate, Evonik Nutrition & Care GmbH), 16,9% VARISOFT EQ 100 (Bis-(Isostearoyl/Oleoyl Isopropyl) Dimonium Methosulfate, Evonik Nutrition & Care GmbH), 1,0% VARISOFT 300 (Cetrimonium Chloride, Evonik Nutrition & Care GmbH), 1,0% REWOTERIC AM C (Sodium Cocoamphoacetate, Evonik Nutrition & Care GmbH) und 13,8% Dipropylenglykol wurden in 28,8% Wasser verrührt. Es bildet sich eine klare Mikroemulsion aus. Der Anteil an signifikant haarkonditionieraktiven Substanzen (TEGOSOFT AC, VARISOFT 300 und VARISOFT EQ 100) beträgt 36,3%.

*Beispiel ME17: Erfindungsgemäße Mikroemulsion:*

17,8% TEGOSOFT AC (Isoamyl Cocoate, Evonik Nutrition & Care GmbH), 19,3% TEGOSOFT PC 41 (Polyglyceryl-4 Caprate, Evonik Nutrition & Care GmbH), 16,5% VARISOFT EQ 100 (Bis-(Isostearoyl/Oleoyl Isopropyl) Dimonium Methosulfate, Evonik Nutrition & Care GmbH), 1,0% VARISOFT 300 (Cetrimonium Chloride, Evonik Nutrition & Care GmbH), 2,0% ANTIL Soft SC (Sorbitan Sesquicaprylate, Evonik Nutrition & Care GmbH) und 16,5% Dipropylenglykol wurden in 26,9% Wasser verrührt. Es bildet sich eine klare Mikroemulsion aus. Der Anteil an signifikant haarkonditionieraktiven Substanzen (TEGOSOFT AC, VARISOFT 300 und VARISOFT EQ 100) beträgt 35,3%.

*Beispiel ME18: Erfindungsgemäße Mikroemulsion:*

25,5% TEGOSOFT M (Isopropyl Myristate, Evonik Nutrition & Care GmbH), 4,9% TEGOSOFT OER (Oleyl Erucate, Evonik Nutrition & Care GmbH), 12,6% VARISOFT EQ 100 (Bis-(Isostearoyl/Oleoyl Isopropyl) Dimonium Methosulfate, Evonik Nutrition & Care GmbH), 22,8% Dipropylenglykol und 25,4% TEGOSOFT PC 41 (Polyglyceryl-4 Caprate, Evonik Nutrition & Care GmbH) wurden in 8,8% Wasser verrührt. Es bildet sich eine klare Mikroemulsion aus. Der Anteil an signifikant haarkonditionieraktiven Substanzen (TEGOSOFT M, TEGOSOFT OER und VARISOFT EQ 100) beträgt 43%.

*Beispiel ME19: Erfindungsgemäße Mikroemulsion:*

25,3% TEGOSOFT M (Isopropyl Myristate, Evonik Nutrition & Care GmbH), 4,8% Arganöl (Argania Spinosa Oil (Argania Spinosa Kernel Oil), DSM Nutritional Products Ltd.), 12,7% VARISOFT EQ 100 (Bis-(Isostearoyl/Oleoyl Isopropyl) Dimonium Methosulfate, Evonik Nutrition & Care GmbH), 22,9% Dipropylenglykol und 25,4% TEGOSOFT PC 41 (Polyglyceryl-4 Caprate, Evonik Nutrition & Care GmbH) wurden in 8,9% Wasser verrührt. Es bildet sich eine klare Mikroemulsion aus. Der Anteil an signifikant haarkonditionieraktiven Substanzen (TEGOSOFT M, Arganöl und VARISOFT EQ 100) beträgt 42,8%.

*Beispiel ME20: Erfindungsgemäße Mikroemulsion:*

26,7% TEGOSOFT M (Isopropyl Myristate, Evonik Nutrition & Care GmbH), 13,3% VARISOFT EQ F 75 Pellets (70% Distearoylethyl Hydroxyethylmonium Methosulfate; 30% Cetearyl Alcohol, Evonik Nutrition & Care GmbH), 24% Dipropylenglykol und 26,7% TEGOSOFT PC 41 (Polyglyceryl-4 Caprate, Evonik Nutrition & Care GmbH) wurden in 9,3% Wasser verrührt. Es bildet sich eine klare Mikroemulsion aus. Der Anteil an signifikant haarkonditionieraktiven Substanzen (TEGOSOFT M und Distearoylethyl Hydroxyethylmonium Methosulfate) beträgt 36%.

*Beispiel ME21:* Nicht erfindungsgemäße *Mikroemulsion:*

30,0% Siloxan, ein Polysiloxan Multi-T-Quat mit N=350 mit folgender statistischen Formel: $(R_2Me_2SiO_{1/2})_7$ $(Me_2SiO_{1/2})_{338}$ $(MeSiO_{3/2})_5$
mit $R_2$ =

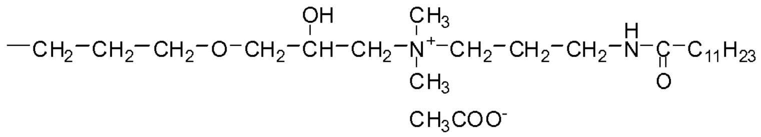

10,0% Propylenglycol, 10,0% TEGOSOFT PC 31 (Polyglyceryl-3 Caprate, Evonik Nutrition & Care GmbH), 5,0% TEGO Betain F 50 (Cocamidopropyl Betaine, 38%-ig, Evonik Nutrition & Care GmbH), 2,4% VARISOFT PATC (Palmitamidopropyltrimonium Chloride, Evonik Nutrition & Care GmbH) wurden in 42,6% Wasser verrührt. Es bildet sich eine klare Mikroemulsion aus. Der Anteil an signifikant haarkonditionieraktiven Substanzen (Siloxan und VARISOFT PATC) beträgt 32,4%.

*Beispiel ME22:* Nicht erfindungsgemäße *Mikroemulsion:*

8% Glyceryl Monooleate (TEGIN O V, Evonik), 25% Dioctylether (Cetiol OE, BASF), 10% Hexadecyl Hexadecanoate (Cutinal CP, BASF), 20,4% C12-16 fatty alcohol 1,4-glucoside (50% in $H_2O$) (Plantacare 1200 UP, BASF), 13,6% C8-16 fatty alcohol 1,4-glucoside (50% in $H_2O$) (Plantacare 2000 UP, BASF), 5,0% Trimethylhexadecylammonium Chloride (CTAC) (Aldrich-Merck), 1,0% Benzoic acid, 2,0% Citric acid wurden in 15,0% Wasser verrührt. Es bildet sich eine klare Mikroemulsion aus. Der Anteil an signifikant haarkonditionieraktiven Substanzen (Dioctylether, Hexadecyl Hexadecanoate und Trimethylhexadecylammonium Chloride) beträgt 40%.

Anwendungstechnische Eigenschaften:

Formulierungsbestandteile sind in den folgenden Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

*Entfernung von Frittiergeruch auf Haaren mittels Haar-Feuchttüchern*

Getestete Formulierungen:

Beispiel A (nicht erfindungsgemäß):

0,67% VARISOFT EQ 100 und 1,33% TEGOSOFT M wurden in Wasser eingerührt. Es bildete sich eine Dispersion aus, die nach kurzem Stehen separiert. D.h. eine homogen Formulierung und damit eine homogene Auftragung auf die Feuchttücher war nicht möglich.

Beispiel B (nicht erfindungsgemäß):

1,33% TEGOSOFT M wurde in Wasser eingerührt. Es bildete sich eine Dispersion aus, die nach kurzem Stehen separiert. D.h. eine homogen Formulierung und Auftragung auf die Feuchttücher war nicht möglich.

Beispiel C (nicht erfindungsgemäß):

0,67% VARISOFT EQ 100 wurde in Wasser dispergiert. Es bildete sich eine leicht trübe Dispersion aus.

Beispiel D (erfindungsgemäß):

5% des Beispiels ME2 wurden in Wasser dispergiert und es bildete sich eine homogene, sehr leicht opake Lösung aus.

Beispiel E (nicht erfindungsgemäß):

10% Plantasil Micro (Beispiel ME4) wurde in Wasser eingerührt. Es bildete sich eine homogene klare Lösung aus.

Behandlung der Haare:

Für die anwendungstechnische Beurteilung wurden Haartressen (4 g, Kaukasisches Haar, Fa. Kerling, Deutschland) durch eine Bleichbehandlung standardisiert vorgeschädigt. Dazu wurden friseurübliche Produkte verwendet. Der Testablauf, die verwendeten Basismaterialien sowie die Details der Beurteilungskriterien sind in DE 103 27 871 beschrieben.

Die Haarsträhnen wurden mit einer SLES-Lösung (12% Sodium Laureth Sulfate in Wasser) vorgewaschen und über Nacht in einem Klimaraum bei 50% Luftfeuchte und 22 °C getrocknet. Eine Fritteuse (Tristar FR-6935) wurde mit 2 l Frittieröl (Palmin) auf 170 °C eingestellt und 750 g Pommes Frites (Mc Cain, 1-2-3-Frites Original) für 10 Minuten gebräunt. Während des Frittiervorgangs wurden die Haarsträhnen in einem Abstand von 20 cm über die Fritteuse gehängt, so dass diese den Geruch des Frittierens aufnehmen.

Feuchttücher:

Feuchttücher (Sontara Style 8838, Woodpulp/Polyester, 20 x 20 cm, 50,8 g/m$^2$, American FlexPack, Inc., Green Bay, WI, USA) wurden mit 5 ml der Formulierungslösung beträufelt und in einem geschlossenen Frischhaltebeutel für 30 min bei 25 °C waagerecht liegend aufbewahrt, um eine gleichmäßige Verteilung der Inhaltsstoffe auf dem Flies zu gewährleisten.

Es wurde daraufhin dreimal mit dem Feuchttuch von oben nach unten über die Haarsträhne gewischt (dabei wurde die Haartresse zwischen dem Feuchttuch geführt). Die Haarsträhnen wurden für 1 h im Klimaraum (22 °C, 50% Luftfeuchte) getrocknet und anschließend in einem Panel-Test bewertet.

Panel-Test:

Die Haarsträhnen wurden durch ein trainiertes Panel mit 10 Personen auf einer Scala von 1 bis 5 (5=am besten) auf Geruch, Gefühl (Weichheit und Geschmeidigkeit der Haare) und Aussehen (Nicht-Fettigkeit) bewertet. In folgender Tabelle sind die Mittelwerte der erhaltenen Panel-Bewertungen angegeben. Je Formulierung wurden jeweils 5 Haarsträhnen bewertet.

| | Blindwert (demin. Wasser) | Beispiel C (nicht erfindungsgemäß) | Beispiel D (erfindungsgemäß) | Beispiel E (nicht erfindungsgemäß) |
|---|---|---|---|---|
| Geruch | 1,0 | 3,0 | 3,9 | 2,2 |
| Gefühl | 1,4 | 4,2 | 5,0 | 3,1 |
| Aussehen | 1,0 | 3,1 | 4,1 | 3,3 |

Deutlich zeigt sich, dass die erfindungsgemäße Formulierung D (mit der Mikroemulsion ME2 mit VARISOFT EQ 100 und TEGOSOFT M) bessere Panelergebnisse in allen drei Bewertungskriterien gibt als die nicht erfindungsgemäße Formulierung C (nur mit VARISOFT EQ 100) und die nicht erfindungsgemäße Formulierung E mit der Mikroemulsion aus dem Markt. Besonders das sehr gute Ergebnis bei der Geruchsentfernung ist hierbei überraschend.

*Schutz vor Aufziehen von Gerüchen auf Haaren*

Haarsträhnen, die wie oben beschrieben mit einer SLES-Lösung vorgewaschen wurden, wurden mit den Feuchttüchern aus Anwendungsbeispiel 1 und wie dort beschrieben behandelt und anschließend wie in Beispiel 1 über eine Fritteuse gehängt. Im Anschluss an den Frittiervorgang wurde direkt der Geruch der Haare bewertet.

| | Blindwert Wasser | Beispiel C (nicht erfindungsgemäß) | Beispiel D (erfindungsgemäß) | Beispiel E (nicht erfindungsgemäß) |
|---|---|---|---|---|
| Geruch | 1,0 | 1,7 | 2,8 | 1,5 |

Das Ergebnis zeigt, dass die erfindungsgemäße Formulierung D auf dem Haar auch einen deutlich schützenden Effekt bzgl. des Aufziehens von Frittiergeruch ausgeübt hat.

*Schnelltrocknung von Haaren*

Haartressen (4 g, Kaukasisches Haar, Fa. Kerling, Deutschland), die durch eine Bleichbehandlung standardisiert vorgeschädigt wurden, wurden mit einer Sodium Laureth Sulfate / Cocamidopropyl Betaine-Lösung (9% : 3%) vorgewaschen und über Nacht im Klimaraum bei 50% Luftfeuchtigkeit und 22 °C getrocknet und am nächsten Tag gewogen ($m_1$).

Die Strähnen wurden zur Messung mit Wasser (38°C) für zehn Sekunden nassgemacht und erneut mit 1 g der Sodium Laureth Sulfate / Cocamidopropyl Betaine-Lösung (9% : 3%) vorgewaschen, indem die Tensidlösung für 1 Minute einmassiert wurde. Anschließend wurden die Haare für 1 Minute mit Wasser (38°C) ausgespült.

Die nassen Haare wurden in eine Schale gelegt und es wurde aus einem Zerstäuber die zu testenden Formulierungen in verdünnter Form aufgesprüht. Es wurde 1 g der verdünnten Formulierung aufgesprüht; die Tresse wurde regelmäßig in der Wanne gewendet, um eine gleichmäßige Bedeckung zu erreichen. Anschließend wurde 1 Minute gewartet und überschüssiges Wasser zweimal zwischen zwei Fingern gleichmäßig von oben nach unten ausgedrückt. Die Haarsträhne wurde erneut gewogen ($m_2$).

Die Haare wurden anschließend erneut mit 1 g der Sodium Laureth Sulfate / Cocamidopropyl Betaine-Lösung (9% : 3%) für 1 Minute gewaschen, 1 Minute mit Wasser ausgespült und 1 Minute abtropfen lassen, ohne die Haare erneut mit den Formulierungen zu behandeln. Die Haare wurden wie beschrieben ausgedrückt und erneut gewogen ($m_3$).

Pro Formulierung wurden 10 Haartressen getestet und der Mittelwert bestimmt.

Der Wassergehalt der Haare nach der Applikation der Formulierungen errechnet sich aus der Differenz $m_2$-$m_1$ und beschreibt den Effekt der Applikation der Formulierung auf den Wassergehalt der Haare.

Der Wassergehalt der Haare nach Applikation und erneutem Waschen der Haare errechnet sich aus der Differenz $m_3$-$m_1$ und zeigt, ob die Formulierung auch nach dem erneuten Waschen noch einen Effekt auf den Wassergehalt zeigt.

Beispiel F (erfindungsgemäß): 1,25% des Beispiels ME2 in Wasser

Beispiel G (erfindungsgemäß): 2,5% des Beispiels ME2 in Wasser

Beispiel H (nicht erfindungsgemäß): 2,5% Plantasil Micro (Beispiel ME4; Dicaprylyl Ether (and) Decyl Glucoside (and) Glyceryl Oleate, BASF) in Wasser

Beispiel I (nicht erfindungsgemäß): 2,5% Lamesoft OD (Beispiel ME5; Coco-Caprylate (and) Lauryl Glucoside (and) Glycerin (and) Polyglyceryl-2 Dipolyhydroxystearate (and) Polyglyceryl-3 Diisostearate, BASF) in Wasser

Wie in Abbildung 1 gezeigt, zeigten die Formulierungen Bespiel F und G mit der erfindungsgemäßen Mikroemulsion Beispiel ME2 überraschenderweise eine deutlich niedrigere Wasseraufnahme als die Vergleichsbeispiele. Dies ist sowohl direkt nach Anwendung der jeweiligen wässrigen Formulierung messbar als auch nach einer anschließenden Wäsche mit einer Tensidformulierung. Die Haare lassen sich daher nach der Anwendung der erfindungsgemäßen Formulierungen schneller trocknen.

*Austestung der Konditionierung von Haaren mittels Sensoriktest - Shampooanwendung 1*

Für die anwendungstechnische Beurteilung der Konditionierung von Haaren wurden die erfindungsgemäßen Mikroemulsionen Beispiel ME1, ME2 und ME3 sowie das Vergleichsbeispiel ME4 Plantasil Micro in einer einfachen kosmetischen Shampooformulierung (Tab. 1) getestet. Es wurden jeweils ca. 1% Aktivstoff (d.h. die Summe aller hauptsächlich konditionieraktiven Substanzen eingesetzt.

Tab. 1: Shampooformulierungen zur Austestung der haarkonditionierenden Eigenschaften. ME1 und ME3 sind nicht erfindungsgemäß

| **Formulierungsbeispiele** | **0a** | **1a** | **2a** | **3a** | **V4a** |
|---|---|---|---|---|---|
| Texapon NSO, 28%-ig, BASF, (INCI: Sodium Laureth Sulfate) | 32% | 32% | 32% | 32% | 32% |
| TEGO Betain F 50, 38%-ig, Evonik Nutrition & Care GmbH, (INCI: Cocamidopropyl Betaine) | 8% | 8% | 8% | 8% | 8% |
| UCARE Polymer JR-400, Dow Chemicals, (INCI: Polyquaternium-10) | 0,3% | 0,3% | 0,3% | 0,3% | 0,3% |

(fortgesetzt)

| Formulierungsbeispiele | 0a | 1a | 2a | 3a | V4a |
|---|---|---|---|---|---|
| ANTIL 171, Evonik Nutrition & Care GmbH, (INCI: PEG-18 Glyceryl Oleate/Cocoate) | 2,5% | 2,5% | 2,5% | 2,5% | 2,5% |
| NaCl | 0,2% | 0,2% | 0,2% | 0,2% | 0,2% |
| Wasser, demineralisiert | ad 100,0% | | | | |
| Zitronensäure (10%-ige Lösung in Wasser) | ad pH 5,5 ± 0,3 | | | | |
| Mikroemulsion Beispiel ME1 (erfindungsgemäß) | - | 2,7 | - | - | - |
| Mikroemulsion Beispiel ME2 (erfindungsgemäß) | - | - | 2,5 | - | - |
| Mikroemulsion Beispiel ME3 (erfindungsgemäß) | - | - | - | 3,6 | - |
| Vergleichsbeispiel ME4 - Plantasil Micro (nicht erfindungsgemäß) | - | - | - | - | 3,6 |
| VISKOSITÄT (Brookfield, 25 °C, in mPa·s) | 6100 | 4000 | 3400 | 3600 | 40 |

Es fällt auf, dass mit den erfindungsgemäßen Formulierungen 1a, 2a und 3a im Gegensatz zur Vergleichsformulierung V4a überraschenderweise wesentlich höhere Viskositäten im Shampoo erreicht werden können.

Die, wie bereits oben in ersten Anwendungsbeispiel ("Entfernung von Frittiergeruch auf Haaren mittels Haar-Feuchttüchern") beschrieben, durch Bleichung vorgeschädigten Haartressen wurden wie folgt mit den Shampooformulierungen behandelt: Die Haartressen wurden unter fließendem, warmem Wasser benetzt. Das überschüssige Wasser wurde leicht von Hand ausgedrückt, dann wurde das Shampoo aufgebracht und sanft im Haar eingearbeitet (1 ml/Haartresse (2 g)). Nach einer Verweilzeit von 1 min wurde das Haar für 1 min mit warmen Wasser (38 °C) ausgespült. Das überschüssige Wasser wurde wieder leicht von Hand ausgedrückt und die Nass-Sensorik durchgeführt. Vor der sensorischen Beurteilung der trockenen Haare wurden die Tressen an der Luft bei 50% Luftfeuchtigkeit und 25 °C für mindestens 12 h getrocknet.

Beurteilungskriterien: Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung. Die Testkriterien sind: Nasskämmbarkeit, Nassgriff, Trockenkämmbarkeit, Trockengriff, Aussehen/Glanz.

In der folgenden Tabelle werden die Ergebnisse der sensorischen Beurteilung der wie oben beschrieben durchgeführten Behandlung der Haarsträhnen verglichen. Das Panel bestand aus 5 trainierten Personen.

Tab. 2: Sensorik-Ergebnisse der Konditionierung von Haaren aus Shampooformulierung

| | Nasskämmbarkeit | Nassgriff | Trockenkämmbarkeit | Trockengriff | Glanz |
|---|---|---|---|---|---|
| Kontrollformulierung 0a (Placebo) | 3,1 | 2,8 | 4,0 | 3,8 | 3,0 |
| Erfindungsgemäße Formulierung 1a | 4,0 | 4,0 | 4,1 | 4,0 | 3,4 |
| Erfindungsgemäße Formulierung 2a | 4,3 | 4,2 | 4,3 | 4,1 | 4,0 |
| Erfindungsgemäße Formulierung 3a | 3,8 | 3,6 | 4,1 | 3,9 | 3,5 |
| Vergleichsformulierung V4a (nicht erfindungsgemäß) | 3,5 | 3,5 | 4,0 | 3,7 | 3,1 |

Die Ergebnisse zeigen in überraschender Weise, dass besonders die erfindungsgemäße Formulierung 2a mit der erfindungsgemäßen Mikroemulsion ME2 signifikant bessere Bewertungen erhält als die Vergleichsformulierung V4a mit dem Vergleichsbeispiel ME4 nach Stand der Technik. Besonders deutlich ist die gute Bewertung der Glanzeigenschaften aller erfindungsgemäßen Formulierungen hervorzuheben.

*Austestung der Konditionierung von Haaren mittels Sensoriktest - Konditioneranwendung*

Für die anwendungstechnische Beurteilung der Konditionierung von Haaren wurde die erfindungsgemäße Mikroemulsion Beispiel ME2 auch in einer einfachen kosmetischen Konditionerformulierung (Tab. 3) getestet. Zum Vergleich wurden die beiden konditionieraktiven Inhaltsstoffe aus ME2, d.h. das Isopropyl Myristate und das Esterquat, auch einzeln (und nicht in Form einer Mikroemulsion) in einer Formulierung eingearbeitet. Es wurde jeweils 1% Aktivstoff (d.h. die Summe aller hauptsächlich konditionieraktiven Substanzen) eingesetzt.

Tab. 3: Konditionerformulierungen zur Austestung der haarkonditionierenden Eigenschaften.

| **Formulierungsbeispiele** | **0b** | **1b** | **V2b** |
|---|---|---|---|
| TEGO Alkanol 1618, Evonik Nutrition & Care GmbH, (INCI: Cetearyl Alcohol) | 7% | 7% | 7% |
| TEGINACID C, Evonik Nutrition & Care GmbH, (INCI: Ceteareth-25) | 1% | 1% | 1% |
| Mikroemulsion Beispiel ME2 (erfindungsgemäß) | - | 2,5% | - |
| VARISOFT EQ 100, Evonik Nutrition & Care GmbH, (INCI: Bis-(Isostearoyl/ Oleoyl Isopropyl) Dimonium Methosulfate) | - | - | 0,33% |
| TEGOSOFT M, Evonik Nutrition & Care GmbH, (INCI: Isopropyl Myristate) | - | - | 0,67% |
| Wasser, demineralisiert | ad 100,00% | | |
| Zitronensäure (10%-ige Lösung in Wasser) | ad pH 4.0 $\pm$ 0,2 | | |
| VISKOSITÄT (Brookfield, 25 °C, in mPa·s) | 14000 | 13900 | 6000 |

Die erfindungsgemäße Formulierung 1b weißt im Gegensatz zur Vergleichsformulierung V2b überraschenderweise eine wesentlich höhere Viskosität im Konditioner auf (trotz der zusätzlichen Bestandteile in der Mikroemulsion).

Die, wie bereits oben in ersten Anwendungsbeispiel beschrieben, durch Bleichung vorgeschädigten und vorgewaschenen Haartressen wurden wie folgt mit den Konditionerformulierungen behandelt: Die Haartressen wurden unter fließendem, warmem Wasser benetzt. Das überschüssige Wasser wurde leicht von Hand ausgedrückt, dann wurde der Konditioner aufgebracht und sanft im Haar eingearbeitet (1 ml/Haartresse (2 g)). Nach einer Verweilzeit von 1 min wurde das Haar für 3 min mit warmen Wasser (38 °C) ausgespült. Das überschüssige Wasser wurde wieder leicht von Hand ausgedrückt und die Nass-Sensorik durchgeführt.

Beurteilungskriterien: Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung. Die Testkriterien sind: Entwirrbarkeit der Haare, Nasskämmbarkeit, Nassgriff.

In der folgenden Tabelle 4 werden die Ergebnisse der sensorischen Beurteilung der wie oben beschrieben durchgeführten Behandlung der Haarsträhnen verglichen. Das Panel bestand aus 4 trainierten Personen.

Tab. 4: Sensorik-Ergebnisse der Konditionierung von Haaren aus Konditionerformulierung

| | Entwirrbarkeit | Nasskämmbarkeit | Nassgriff |
|---|---|---|---|
| Kontrollformulierung 0b (Placebo) | 2,0 | 1,8 | 1,6 |
| Erfindungsgemäße Formulierung 1b | 3,6 | 3,8 | 4,2 |
| Vergleichsformulierung V2b (nicht erfindungsgemäß) | 3,3 | 3,3 | 3,5 |

Die Ergebnisse zeigen in überraschender Weise, dass die erfindungsgemäße Formulierung 1b mit der erfindungsgemäßen Mikroemulsion ME2 sehr viel bessere Bewertungen erhält als die Vergleichsformulierung V2b, in der die konditionieraktiven Substanzen separat eingearbeitet wurden.

*Austestung der Konditionierung von Haaren mittels Sensoriktest - Shampooanwendung 2*

Für die anwendungstechnische Beurteilung der Konditionierung von Haaren wurde die erfindungsgemäße Mikroemulsion Beispiel ME2, ME 20, ME21 und ME22 in einer einfachen kosmetischen Shampooformulierung (Tab. 5) getestet.

Tab. 5: Shampooformulierungen zur Austestung der haarkonditionierenden Eigenschaften.

| **Formulierungsbeispiele** | **0c** | **1c** | **2c** | **3c** | **4c** |
|---|---|---|---|---|---|
| Texapon NSO, 28%-ig, BASF, (INCI: Sodium Laureth Sulfate) | 32% | 32% | 32% | 32% | 32% |
| TEGO Betain F 50, 38%-ig, Evonik Nutrition & Care GmbH, (INCI: Cocamidopropyl Betaine) | 8% | 8% | 8% | 8% | 8% |
| UCARE Polymer JR-400, Dow Chemicals, (INCI: Polyquaternium-10) | 0,3% | 0,3% | 0,3% | 0,3% | 0,3% |
| ANTIL 171, Evonik Nutrition & Care GmbH, (INCI: PEG-18 Glyceryl Oleate/Cocoate) | 2,5% | 2,5% | 2,5% | 2,5% | 2,5% |
| NaCl | 0,2% | 0,2% | 0,2% | 0,2% | 0,2% |
| Wasser, demineralisiert | ad 100,0% | | | | |
| Zitronensäure (10%-ige Lösung in Wasser) | ad pH 5,5 ± 0,3 | | | | |
| Mikroemulsion Beispiel ME20 (erfindungsgemäß) | - | 2,8 | - | - | - |
| Mikroemulsion Beispiel ME2 (erfindungsgemäß) | - | - | 2,5 | - | - |
| Mikroemulsion Beispiel ME22 (erfindungsgemäß) | - | - | - | 2,5 | - |
| Mikroemulsion Beispiel ME21 (erfindungsgemäß) | - | - | - | - | 3,1 |
| VISKOSITÄT (Brookfield, 25 °C, in mPa·s) | 6000 | 1500 | 2900 | 540 | 110 |

Die Austestung der Formulierungen aus Tabelle 5 erfolgt ebenfalls genauso wie bereits oben für Tabelle 2 beschrieben:

Tab. 6: Sensorik-Ergebnisse der Konditionierung von Haaren aus Shampooformulierung.

| | Nasskämmbarkeit | Nassgriff | Trockenkämmbarkeit | Trockengriff | Glanz |
|---|---|---|---|---|---|
| Kontrollformulierung 0c (Placebo) | 3,0 | 3,0 | 4,0 | 3,8 | 3,2 |
| Erfindungsgemäße Formulierung 1c | 4,0 | 4,0 | 4,2 | 4,0 | 3,6 |
| Erfindungsgemäße Formulierung 2c | 4,3 | 4,3 | 4,3 | 4,2 | 3,9 |
| Erfindungsgemäße Formulierung 3c | 3,6 | 3,5 | 4,0 | 3,9 | 3,3 |
| Erfindungsgemäße Formulierung 4c | 4,4 | 4,5 | 4,3 | 3,7 | 3,9 |

Es fällt hier auf, dass die erfindungsgemäße Formulierung 2c ähnlich sehr gute Werte aufweist wie die zusätzlich mit Siliconquat versehene Formulierung 4c und zusätzlich vor allem im Trockengriff die Siliconquat-Formulierung deutlich übertrifft.

Für die Formulierungen 1c und 2c fallen die guten Viskositäten auf; die Formulierungen benötigen somit wenig rheologische Additive und Verdickungsmittel, um auf eine für Shampoos übliche Viskosität zu kommen, sie lassen sich also sehr gut verdicken.

*Weitere Formulierungsbeispiele:*

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen

Tabellen gekennzeichnet. Bei dreiphasigen Prozessen werden die drei Phasen mit A, B und C benannt. Wenn nicht anders angegeben handelt es sich bei den Angaben in den folgenden Tabellen um Angaben in Gew.-%.

Formulierungsbeispiel 1) Clear Shampoo (Nicht erfindungsgemäß)

| | |
|---|---|
| TEXAPON@ NSO, BASF, 28%-ig (INCI: Sodium Laureth Sulfate) | 32,00% |
| Mikroemulsion ME3 | 2,00% |
| Perfume | 0,20% |
| TEGO@ Betain F 50, Evonik Nutrition & Care GmbH, 38%-ig (INCI: Cocamidopropyl Betaine) | 8,00% |
| ANTIL® 171 Evonik Nutrition & Care GmbH, (INCI: PEG-18 Glyceryl Oleate/Cocoate) | 1,70% |
| Water | ad 100,00% |
| NaCl | 0,80% |
| Citric Acid, 30%-ig | q.s. to pH 5,5 |
| Preservative | q.s. |

Formulierungsbeispiel 2) Clear Conditioning Shampoo (Nicht erfindungsgemäß)

| | |
|---|---|
| TEXAPON@ NSO, BASF, 28%-ig (INCI: Sodium Laureth Sulfate) | 35,00% |
| ANTIL® 200, Evonik Nutrition & Care GmbH (INCI: PEG-200 Hydrogenated Glyceryl Palmate; PEG-7 Glyceryl Cocoate) | 1,50% |
| Mikroemulsion ME1 | 2,00% |
| Perfume | 0,25% |
| Water | ad 100,00% |
| Polymer JR 400, Amerchol, (INCI: Polyquaternium-10) | 0,20% |
| TEGO@ Betain F 50, Evonik Nutrition & Care GmbH, 38%-ig (INCI: Cocamidopropyl Betaine) | 8,00% |
| NaCl | 1,50% |
| Citric Acid, 30 %-ig | q.s. to pH 5,5 |
| Preservative | q.s. |

Formulierungsbeispiel 3) Clear Conditioning Shampoo

| | |
|---|---|
| TEXAPON@ NSO, BASF, 28%-ig (INCI: Sodium Laureth Sulfate) | 20,00% |
| ANTIL® 200, Evonik Nutrition & Care GmbH, (INCI: PEG-200 Hydrogenated Glyceryl Palmate; PEG-7 Glyceryl Cocoate) | 1,80% |
| ABIL® Quat 3272, Evonik Nutrition & Care GmbH, (INCI: Quaternium-80) | 1,00% |
| Mikroemulsion ME2 | 2,50% |
| Perfume | 0,25% |
| Water | ad 100,00% |
| Polymer JR 400, Amerchol (INCI: Polyquaternium-10) | 0,30% |

(fortgesetzt)

| | |
|---|---|
| TEGO@ Betain F 50, Evonik Nutrition & Care GmbH, 38%-ig (INCI: Cocamidopropyl Betaine) | 18,00% |
| NaCl | 0,90% |
| Lactic Acid, 80%-ig | q.s. to pH 5,5 |
| Preservative | q.s. |

Formulierungsbeispiel 4) Clear Conditioning Shampoo

| | |
|---|---|
| TEXAPON@ NSO, BASF, 28%-ig (INCI: Sodium Laureth Sulfate) | 32,00% |
| ANTIL® 500 Pellets, Evonik Nutrition & Care GmbH (INCI: PEG-200 Glyceryl Stearate) | 1,00% |
| ABIL® B 8832, Evonik Nutrition & Care GmbH (INCI: Bis-PEG/PPG-20/20 Dimethicone) | 0,50% |
| Mikroemulsion ME6 | 3,50% |
| Perfume | 0,20% |
| Water | ad 100,00% |
| Polymer JR 400, Amerchol (INCI: Polyquaternium-10) | 0,20% |
| TEGO@ Betain F 50, Evonik Nutrition & Care GmbH, 38%-ig (INCI: Cocamidopropyl Betaine) | 8,00% |
| NaCl | 0,80% |
| Citric Acid, 30%-ig | q.s. to pH 5,5 |
| Preservative | q.s. |

Formulierungsbeispiel 5) Clear Conditioning Shampoo

| | |
|---|---|
| TEXAPON@ NSO, BASF, 28%-ig (INCI: Sodium Laureth Sulfate) | 32,00% |
| VARISOFT® PATC, Evonik Nutrition & Care GmbH, (INCI: Palmitamidopropyltrimonium Chloride) | 1,50% |
| REWODERM@ LI S 80, Evonik Nutrition & Care GmbH, (INCI: PEG-200 Hydrogenated Glyceryl Palmate; PEG-7 Glyceryl Cocoate) | 2,50% |
| Mikroemulsion ME13 | 2,00% |
| Perfume | 0,15% |
| Water | ad 100,00% |
| TEGO@ Cosmo C 100, Evonik Nutrition & Care GmbH, (INCI: Creatine) | 1,00% |
| Jaguar C-162, Solvay, (INCI: Hydroxypropyl Guar Hydroxypropyltrimonium Chloride) | 0,30% |
| TEGO@ Betain F 50, Evonik Nutrition & Care GmbH, 38%-ig (INCI: Cocamidopropyl Betaine) | 8,00% |
| NaCl | 1,50% |
| Citric Acid, 30%-ig | q.s. to pH 5,5 |
| Preservative | q.s. |

Formulierungsbeispiel 6) Clear Conditioning Shampoo

| | |
|---|---|
| Mikroemulsion ME16 | 2,50% |
| Argan Oil, DSM Nutritional Products Ltd., (INCI: Argania Spinosa Oil (Argania Spinosa Kernel Oil)) | 0,1% |
| TEXAPON@ NSO, BASF, 28%-ig (INCI: Sodium Laureth Sulfate) | 32,00% |
| REWODERM@ LI S 80, Evonik Nutrition & Care GmbH, (INCI: PEG-200 Hydrogenated Glyceryl Palmate; PEG-7 Glyceryl Cocoate) | 2,00% |
| Perfume | 0,25% |
| Water | ad 100,00% |
| TEGO@ Cosmo C 100, Evonik Nutrition & Care GmbH, (INCI: Creatine) | 1,50% |
| Jaguar C-162, Solvay, (INCI: Hydroxypropyl Guar Hydroxypropyltrimonium Chloride) | 0,20% |
| TEGO@ Betain F 50, Evonik Nutrition & Care GmbH, 38%-ig | 8,00% |
| (INCI: Cocamidopropyl Betaine) | |
| NaCl | 2,50% |
| Citric Acid, 30%-ig | q.s. to pH 5,0 |
| Preservative | q.s. |

Formulierungsbeispiel 7) Pearlized Shampoo

| | |
|---|---|
| TEXAPON@ NSO, BASF, 28%-ig (INCI: Sodium Laureth Sulfate) | 32,00% |
| Mikroemulsion ME19 | 5,50% |
| Perfume | 0,15% |
| Water | ad 100,00% |
| TEGO@ Betain F 50, Evonik Nutrition & Care GmbH, 38%-ig (INCI: Cocamidopropyl Betaine) | 8,00% |
| TEGO® Pearl N 300, Evonik Nutrition & Care GmbH, (INCI: Glycol Distearate; Laureth-4; Cocamidopropyl Betaine) | 2,00% |
| ANTIL® 171 Evonik Nutrition & Care GmbH, (INCI: PEG-18 Glyceryl Oleate/Cocoate) | 2,50% |
| NaCl | 0,90% |
| Citric Acid, 30%-ig | q.s. to pH 5,5 |
| Preservative | q.s. |

Formulierungsbeispiel 8) Turbid Conditioning Shampoo

| | |
|---|---|
| TEXAPON@ NSO, BASF, 28%-ig (INCI: Sodium Laureth Sulfate) | 32,00% |
| ANTIL® 200, Evonik Nutrition & Care GmbH, (INCI: PEG-200 Hydrogenated Glyceryl Palmate; PEG-7 Glyceryl Cocoate) | 2,00% |
| Mikroemulsion ME18 | 1,00% |
| Perfume | 0,25% |
| Water | ad 100,00% |
| Polymer JR 400, Amerchol, (INCI: Polyquaternium-10) | 0,40% |
| TEGO@ Betain F 50, Evonik Nutrition & Care GmbH, 38%-ig (INCI: Cocamidopropyl Betaine) | 8,00% |

(fortgesetzt)

| | |
|---|---|
| DC1503 Fluid, Dow Corning, (INCI: Dimethicone, Dimethiconol) | 1,00% |
| TEGO@ Pearl N 300, Evonik Nutrition & Care GmbH, (INCI: Glycol Distearate; Laureth-4; Cocamidopropyl Betaine) | 2,00% |
| NaCl | 0,30 |
| Citric Acid, 30%-ig | q.s. to pH 5,5 |
| Preservative | q.s. |

Formulierungsbeispiel 9) Schaumiges Körperpflegemittel

| | |
|---|---|
| TEXAPON@ NSO, BASF, 28%-ig (INCI: Sodium Laureth Sulfate) | 15,00% |
| Perfume | 0,30% |
| Mikroemulsion ME7 | 1,50% |
| REWOTERIC® AM C, Evonik Nutrition & Care GmbH, 32%-ig (INCI: Sodium Cocoamphoacetate) | 8,00% |
| Water | ad 100,00% |
| TEGOCEL® HPM 50, Evonik Nutrition & Care GmbH, (INCI: Hydroxypropyl Methylcellulose) | 0,50% |
| LACTIL®, Evonik Nutrition & Care GmbH, (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |
| Preservative | q.s. |

Formulierungsbeispiel 10) Körperpflegemittel

| | |
|---|---|
| TEXAPON@ NSO, BASF, 28%-ig (INCI: Sodium Laureth Sulfate) | 30,00% |
| TEGOSOFT® PC 31, Evonik Nutrition & Care GmbH, (INCI: Polyglyceryl-3 Caprate) | 0,50% |
| Mikroemulsion ME2 | 4,50% |
| Perfume | 0,30% |
| Water | ad 100,00% |
| TEGOCEL® HPM 4000, Evonik Nutrition & Care GmbH, (INCI: Hydroxypropyl Methylcellulose) | 0,30% |
| Jaguar C-162, Solvay, (INCI: Hydroxypropyl Guar Hydroxypropyltrimonium Chloride) | 0,20% |
| REWOTERIC® AM C, Evonik Nutrition & Care GmbH, 32%-ig (INCI: Sodium Cocoamphoacetate) | 12,00% |
| Citric Acid Monohydrate | 0,50% |
| REWODERM@ LI S 80, Evonik Nutrition & Care GmbH, (INCI: PEG-200 Hydrogenated Glyceryl Palmate; PEG-7 Glyceryl Cocoate) | 2,00% |
| TEGO® Pearl N 300, Evonik Nutrition & Care GmbH, (INCI: Glycol Distearate; Laureth-4; Cocamidopropyl Betaine) | 2,00% |
| NaCl | 1,00% |
| Preservative | q.s. |

Formulierungsbeispiel 11) Schaumiges Körperpflegemittel (Nicht erfindungsgemäß)

| | |
|---|---|
| TEXAPON@ NSO, BASF, 28%-ig (INCI: Sodium Laureth Sulfate) | 14,30% |
| ANTIL® 500 Pellets, Evonik Nutrition & Care GmbH, (INCI: PEG-200 Glyceryl Stearate) | 0,70% |
| Perfume | 0,30% |
| Mikroemulsion ME3 | 1,00% |
| REWOTERIC® AM C, Evonik Nutrition & Care GmbH, 32%-ig (INCI: Sodium Cocoamphoacetate) | 10,00% |
| NaCl | 1,50% |
| Water | ad 100,00% |
| Merquat 550, Nalco, (INCI: Polyquaternium-7) | 0,30% |
| LACTIL®, Evonik Nutrition & Care GmbH, (INCI: Sodium Lactate; Sodium PCA; Glycine; Fructose; Urea; Niacinamide; Inositol; Sodium benzoate; Lactic Acid) | 0,50% |
| Citric Acid, 30%-ig | q.s. to pH 5,5 |

Formulierungsbeispiel 12) Mild Foam Bath

| | |
|---|---|
| TEXAPON® NSO, BASF, 28%-ig (INCI: Sodium Laureth Sulfate) | 27,00% |
| REWOPOL® SB FA 30, Evonik Nutrition & Care GmbH, 40%-ig (INCI: Disodium Laureth Sulfosuccinate) | 12,00% |
| TEGOSOFT® LSE 65 K SOFT, Evonik Nutrition & Care GmbH, (INCI: Sucrose Cocoate) | 2,00% |
| Water | ad 100,00% |
| Jaguar C-162, Solvay, (INCI: Hydroxypropyl Guar Hydroxypropyltrimonium Chloride) | 0,15% |
| REWOTERIC® AM C, Evonik Nutrition & Care GmbH, 32%-ig (INCI: Sodium Cocoamphoacetate) | 12,00% |
| Mikroemulsion ME7 | 1,50% |
| Citric Acid, 30%-ig | 3,00% |
| ANTIL® 171, Evonik Nutrition & Care GmbH, (INCI: PEG-18 Glyceryl Oleate/Cocoate) | 1,75% |
| NaCl | 1,00% |
| TEGO® Pearl N 300 Evonik Nutrition & Care GmbH (INCI: Glycol Distearate; Laureth-4; Cocamidopropyl Betaine) | 2,00% |

Formulierungsbeispiel 13) feuchtigkeitspendendes Hautreinigungsmittel

| | | |
|---|---|---|
| A | TEXAPON@ NSO, BASF, 28%-ig, (INCI: Sodium Laureth Sulfate) | 30,00% |
| | Mikroemulsion ME2 | 3,70% |
| | Perfume | 0,30% |

(fortgesetzt)

| | | |
|---|---|---|
| B | Water | ad 100,00% |
| | TEGOCEL® fluid HPM 4000, Evonik Nutrition & Care GmbH, (INCI: Hydroxypropyl Methylcellulose) | 1,20% |
| | TEGO@ Betain C 60, Evonik Nutrition & Care GmbH, 46%-ig, (INCI: Cocamidopropyl Betaine) | 8,10% |
| | TEGOSOFT® APM, Evonik Nutrition & Care GmbH, (INCI: PPG-3 Myristyl Ether) | 1,00% |
| | Cutina TS, BASF (INCI:PEG- 3 Distearate) | 1,00% |
| | REWODERM@ LI S 80, Evonik Nutrition & Care GmbH, (INCI: PEG-200 Hydrogenated Glyceryl Palmate; PEG-7 Glyceryl Cocoate) | 1,50% |
| | Preservative | 0,60% |
| | Citric Acid, 30%-ig | q.s. to pH 5,5 |

Formulierunsbeisiel 14) Duschgel

| | |
|---|---|
| TEXAPON@ NSO, BASF, 28%-ig (INCI: Sodium Laureth Sulfate) | 15,00% |
| Mikroemulsion ME3 | 1,50% |
| Mikroemulsion ME19 | 1,00% |
| Perfume | 0,30% |
| PGFAC-S, BASF, (INCI: Sodium cocoyl hydrolyzed wheat protein glutamate) | 1,00% |
| REWOPOL SB CS 50 B, Evonik Nutrition & Care GmbH, 40%-ig, (INCI: Disodium PEG-5 Laurylcitrate Sulfosuccinate; Sodium Laureth Sulfate) | 7,50% |
| Water | ad 100,00% |
| TEGO@ Betain F 50, Evonik Nutrition & Care GmbH, 38%-ig, (INCI: Cocamidopropyl Betaine) | 9,00% |
| TEGO@ Betain 810, Evonik Nutrition & Care GmbH, 38%-ig, (INCI: Capryl/Capramidopropyl Betaine) | 4,00% |
| Merquat 550, Nalco, (INCI: Polyquaternium-7) | 0,50% |
| ANTIL® 200, Evonik Nutrition & Care GmbH, (INCI: PEG-200 Hydrogenated Glyceryl Palmate; PEG-7 Glyceryl Cocoate) | 2,00% |
| NaCl | 1,80% |
| Citric Acid, 30%-ig | q.s. to pH 5,2 |
| Preservative | q.s. |

Formulierunsbeisiel 15) Körperreinigungsmittel

| | | |
|---|---|---|
| A | TEXAPON® NSO, BASF, 28%-ig, (INCI: Sodium Laureth Sulfate) | 30,00% |
| | Mikroemulsion ME7 | 1,50% |
| | ABIL® B 8832, Evonik Nutrition & Care GmbH, (INCI: Bis-PEG/PPG-20/20 Dimethicone) | 0,30% |
| | Perfume | 0,30% |

(fortgesetzt)

| | | |
|---|---|---|
| B | Water | ad 100,00% |
| | TEGOCEL® fluid HPM 4000, Evonik Nutrition & Care GmbH, (INCI: Hydroxypropyl Methylcellulose) | 1,20% |
| | Jaguar C-162, Solvay, (INCI: Hydroxypropyl Guar Hydroxypropyltrimonium Chloride) | 0,15% |
| | Citric Acid Monohydrate | 0,50% |
| | REWOTERIC® AM C, Evonik Nutrition & Care GmbH, 32%-ig, (INCI: Sodium Cocoamphoacetate) | 10,00% |
| | Cutina TS, BASF, (INCI: PEG- 3 Distearate) | 2,00% |
| | REWODERM@ LI S 80, Evonik Nutrition & Care GmbH, (INCI: PEG-200 Hydrogenated Glyceryl Palmate; PEG-7 Glyceryl Cocoate) | 2,60% |
| C | Preservative | 0,60% |

Formulierungsbeispiel 16) Body Cleansing Foam

| | |
|---|---|
| TEXAPON@ NSO, BASF, 28%-ig (INCI: Sodium Laureth Sulfate) | 18,00% |
| Perfume | 0,30% |
| Mikroemulsion ME18 | 0,70% |
| Cropure Olive, Croda, (INCI: Olea Europaea (Olive) Fruit Oil) | 0,10% |
| REWOTERIC® AM C, Evonik Nutrition & Care GmbH, 32%-ig (INCI: Sodium Cocoamphoacetate) | 8,00% |
| TEGO® Betain F 50, Evonik Nutrition & Care GmbH, 38%-ig (INCI: Cocamidopropyl Betaine) | 8,00% |
| Water | ad 100,00% |
| Jaguar C-162, Solvay, (INCI: Hydroxypropyl Guar Hydroxypropyltrimonium Chloride) | 0,25% |
| ANTIL® Soft SC, Evonik Nutrition & Care GmbH, (INCI: Sorbitan Sesquicaprylate) | 0,50% |
| ANTIL® 120 Plus, Evonik Nutrition & Care GmbH, | 1,50% |
| (INCI: PEG-120 Methyl Glucose Dioleate) | |
| TEGO@ Cosmo C 100, Evonik Nutrition & Care GmbH, (INCI: Creatine) | 1,00% |
| NaCl | 1,00% |
| Panthenol, BASF, (INCI: D- Panthenol) | 0,20% |
| Citric Acid Monohydrate | 0,50% |

Formulierungsbeispiel 17) Anti-Schuppen Shampoo

| | |
|---|---|
| TEXAPON® LS 35, BASF, 30%-ig (INCI: Sodium Lauryl Sulfate) | 24,00% |
| TAGAT® CH 40, Evonik Nutrition & Care GmbH, (INCI: PEG-40 Hydrogenated Castor Oil) | 2,00% |
| TEGOSOFT® GC, Evonik Nutrition & Care GmbH, (INCI: PEG-7 Glyceryl Cocoate) | 1,00% |
| Cropure Avocado, Croda, (INCI: Persea Gratissima (Avocado) Oil) | 0,15% |
| Mikroemulsion ME19 | 2,00% |
| Perfume | 0,20% |

(fortgesetzt)

| | |
|---|---|
| Water | ad 100,00% |
| Polymer JR 400, Amerchol, (INCI: Polyquaternium-10) | 0,20% |
| TEGO® Betain F 50, Evonik Nutrition & Care GmbH, 38%-ig (INCI: Cocamidopropyl Betaine) | 16,00% |
| Hostapon SG, Clariant, (INCI : Sodium Cocoyl Glycinate) | 5,00% |
| Microcare ZP, Thor, (INCI: Zinc Pyrithione) | 0,20% |
| Octopirox, Clariant, (INCI: Octopirox) | 0,10% |
| ABIL® Quat 3272, Evonik Nutrition & Care GmbH, (INCI: Quaternium-80) | 0,80% |
| REWOMID® D 212, Evonik Nutrition & Care GmbH, (INCI: Cocamide MEA) | 0,80% |
| ANTIL® 500, Evonik Nutrition & Care GmbH, (INCI: PEG-200 Glyceryl Stearate) | 0,80% |
| Glycerin | 1,50% |
| NaCl | 0,90% |
| Citric Acid, 30%-ig | q.s. to pH 5,5 |
| Preservative | q.s. |

Formulierungsbeispiel 18) PEG-freies Shampoo

| | |
|---|---|
| TEXAPON® LS 35, BASF, 30%-ig (INCI: Sodium Lauryl Sulfate) | 12,00% |
| TEGO® Betain F 50, Evonik Nutrition & Care GmbH, 38%-ig (INCI: Cocamidopropyl Betaine) | 18,00% |
| Iselux, Innospec, (INCI: Sodium Lauroyl Methyl Isethionate) | 4,00% |
| TEGOSOFT® PC 41, Evonik Nutrition & Care GmbH, (INCI: Polyglyceryl-4 Caprate) | 2,00% |
| Mikroemulsion ME18 | 1,50% |
| Perfume | 0,25% |
| Water | ad 100,00% |
| Jaguar C-162, Solvay (INCI: Hydroxypropyl Guar Hydroxypropyltrimonium Chloride) | 0,20% |
| Versene 100, The Dow Chemical Company, (INCI: Tetrasodium EDTA) | 0,20% |
| REWOMID® D 212, Evonik Nutrition & Care GmbH, (INCI: Cocamide MEA) | 0,80% |
| ANTIL® SPA 80, Evonik Nutrition & Care GmbH, (INCI: Isostearamide MIPA; Glyceryl Laurate) | 0,80% |
| Keltrol CG-SFT, CP Kelco, (INCI: Xanthan Gum) | 0,20% |
| Glycerin | 1,50% |
| NaCl | 0,70% |
| Citric Acid, 30%-ig | q.s. to pH 5,1 |
| Preservative | q.s. |

Formulierungsbeispiel 19) Conditioning Anti-Schuppen Shampoo ( Nicht erfindungsgemäß)

| | | |
|---|---|---|
| A | TEGIN® G 1100 Pellets, Evonik Nutrition & Care GmbH, (INCI: Glycol Distearate) | 3,00% |
| | TEXAPON® NSO, BASF, 28%-ig, (INCI: Sodium Laureth Sulfate) | 40,00% |

(fortgesetzt)

| | | |
|---|---|---|
| B | Perfume | 0,30% |
| | Zinc-Pyrion NF, Wey!Chem, 48%-ig, (INCI: Zinc Pyrithione) | 2,00% |
| | Mikroemulsion ME3 | 2,00% |
| C | Water | ad 100,00% |
| | TEGO® Carbomer 341 ER, Evonik Nutrition & Care GmbH, (INCI: Acrylates/C10-30 Alkyl Acrylate Crosspolymer) | 0,20% |
| | Water | 0,30% |
| | NaOH, 25%-ig | 0,30% |
| D | REWOTERIC® AM B U 185, Evonik Nutrition & Care GmbH, 30%-ig, (INCI: Undecylenamidopropyl Betaine) | 12,50% |
| | ANTIL® SPA 80, Evonik Nutrition & Care GmbH, (INCI: Isostearamide MIPA; Glyceryl Laurate) | 3,70% |
| | Preservative | q.s. |

| | |
|---|---|
| Formulierungsbeispiel 20) Shampoo, PEG- & Sulfat-frei | |
| REWOTERIC® AM C, Evonik Nutrition & Care GmbH, 32%-ig, (INCI: Sodium Cocoamphoacetate) | 16,00% |
| Plantapon ACG 50, BASF, (INCI: Disodium Cocoyl Glutamate) | 3,80% |
| Plantacare® 818 UP, BASF, 51%-ig, (INCI: Coco Glucoside) | 5,00% |
| Mikroemulsion ME2 | 2,50% |
| Perfume | 0,30% |
| Water | ad 100,00% |
| TEGO@ Betain F 50, Evonik Nutrition & Care GmbH, 38%-ig, (INCI: Cocamidopropyl Betaine) | 6,00% |
| ANTIL® SPA 80, Evonik Nutrition & Care GmbH, (INCI: Isostearamide MIPA; Glyceryl Laurate) | 1,80% |
| Preservative | q.s. |
| Citric Acid, 30%-ig | q.s. to pH 5,0 |

Formulierungsbeispiel 21) Shampoo, PEG- & Sulfat-frei

| | | |
|---|---|---|
| A | REWOTERIC® AMC, Evonik Nutrition & Care GmbH, 32%-ig, (INCI: Sodium Cocoamphoacetate) | 20,00% |
| | REWOPOL® SB F 12 P, Evonik Nutrition & Care GmbH, 96%-ig, (INCI: Disodium Lauryl Sulfosuccinate) | 6,00% |
| | Mikroemulsion ME18 | 2,00% |
| | ANTIL® SPA 80, Evonik Nutrition & Care GmbH, (INCI: Isostearamide MIPA; Glyceryl Laurate) | 1,70% |
| B | Water | ad 100,00% |
| | Jaguar C-162, Solvay, (INCI: Hydroxypropyl Guar Hydroxypropyltrimonium Chloride) | 0,10% |
| | Citric Acid, 30%-ig | 3,60% |

(fortgesetzt)

| | | |
|---|---|---|
| C | ANTIL® HS 60, Evonik Nutrition & Care GmbH, (INCI: Cocamidopropyl Betaine; Glyceryl Laurate) | 3,00% |
| | Preservative | q.s. |

Formulierungsbeispiel 22) Mild Hair & Body Wash, PEG- und Sulfat-frei (Nicht erfindungsgemäß)

| | |
|---|---|
| Plantacare® 1200 UP, BASF, 50%-ig, (INCI: Lauryl Glucoside) | 11,40% |
| Plantacare® 818 UP, BASF, 51%-ig, (INCI: Coco Glucoside) | 5,60% |
| Water | ad 100,00% |
| ANTIL® Soft SC, Evonik Nutrition & Care GmbH, (INCI: Sorbitan Sesquicaprylate) | 0,90% |
| Mikroemulsion ME3 | 1,00% |
| TEGOSOFT® LSE 65 K SOFT, Evonik Nutrition & Care GmbH, (INCI: Sucrose Cocoate) | 1,50% |
| TEGO@ Betain F 50, Evonik Nutrition & Care GmbH, 38%-ig, (INCI: Cocamidopropyl Betaine) | 18,00% |
| Perfume, preservative | q.s. |
| Citric Acid, 30%-ig | q.s. to pH 5.0 |

Formulierungsbeispiel 23) Mild Shampoo, Sulfat-frei (Nicht erfindungsgemäß)

| | |
|---|---|
| Plantacare® 1200 UP, BASF, 50%-ig, (INCI: Lauryl Glucoside) | 8,50% |
| Plantacare® 818 UP, BASF, 51%-ig, (INCI: Coco Glucoside) | 2,30% |
| Water | ad 100,00% |
| ANTIL® 200, Evonik Nutrition & Care GmbH, (INCI: PEG-200 Hydrogenated Glyceryl Palmate; PEG-7 Glyceryl Cocoate) | 4,00% |
| Mikroemulsion ME3 | 5,00% |
| REWOPOL® SB FA 30 B, Evonik Nutrition & Care GmbH, (INCI: Disodium Laureth Sulfosuccinate) | 9,00% |
| TEGO@ Betain F 50, Evonik Nutrition & Care GmbH, 38%-ig, (INCI: Cocamidopropyl Betaine) | 14,70% |
| Perfume, preservative | q.s. |
| Citric Acid, 30%-ig | q.s. to pH 5.0 |

Formulierungsbeispiel 24) Mild Shampoo, Sulfat-frei (Nicht erfindungsgemäß)

| | |
|---|---|
| Plantacare® 1200 UP, BASF, 50%-ig, (INCI: Lauryl Glucoside) | 8,50% |
| Plantacare® 818 UP, BASF, 51%-ig, (INCI: Coco Glucoside) | 2,30% |
| Water | ad 100,00% |
| Tilamar 740, DSM, (INCI: Polyquaternium-7) | 0,20% |
| ANTIL® 171, Evonik Nutrition & Care GmbH, (INCI: PEG-18 Glyceryl Oleate/Cocoate) | 0,60% |
| Mikroemulsion ME3 | 3,00% |

(fortgesetzt)

| | |
|---|---|
| Perlastan SC 25 NKW, Schill&Seilacher, (INCI: Disodium Cocoyl Glutamate, Sodium Cocoyl Glutamate) | 14,40% |
| TEGO@ Betain F 50, Evonik Nutrition & Care GmbH, 38%-ig, (INCI: Cocamidopropyl Betaine) | 14,70% |
| Perfume, preservative | q.s. |
| Citric Acid, 30%-ig | q.s. to pH 5.3 |

Formulierungsbeispiel 25 Mild Shampoo, PEG- und Sulfat-frei

| | |
|---|---|
| Plantacare® 1200 UP, BASF, 50%-ig, (INCI: Lauryl Glucoside) | 10,00% |
| Water | ad 100,00% |
| UCARE Polymer JR-400, Dow Chemicals, (INCI: Polyquaternium-10) | 0,10% |
| ANTIL® SPA 80, Evonik Nutrition & Care GmbH, (INCI: Isostearamide MIPA; Glyceryl Laurate) | 1,20% |
| Mikroemulsion ME2 | 2,50% |
| REWOTERIC® AM C, Evonik Nutrition & Care GmbH, 32%-ig, (INCI: Sodium Cocoamphoacetate) | 21,30% |
| TEGO@ Betain P 50 C, Evonik Nutrition & Care GmbH, 38%-ig, (INCI: Cocamidopropyl Betaine) | 10,50% |
| Perfume, preservative | q.s. |
| Citric Acid, 30%-ig | q.s. to pH 5.1 |

Formulierungsbeispiel 26) Shampoo (Nicht erfindungsgemäß)

| | |
|---|---|
| TEXAPON® NSO, BASF, 28%-ig, (INCI: Sodium Laureth Sulfate) | 32,10% |
| Water | ad 100,00% |
| UCARE Polymer JR-400, Dow Chemicals, (INCI: Polyquaternium-10) | 0,20% |
| ANTIL® SPA 80, Evonik Nutrition & Care GmbH, (INCI: Isostearamide MIPA; Glyceryl Laurate) | 0,50% |
| ANTIL® 171, Evonik Nutrition & Care GmbH, (INCI: PEG-18 Glyceryl Oleate/Cocoate) | 2,00% |
| Mikroemulsion ME3 | 5,00% |
| TEGO® Pearl N 300, Evonik Nutrition & Care GmbH, (INCI: Glycol Distearate; Laureth-4; Cocamidopropyl Betaine) | 1,50% |
| TEGO@ Betain F 50, Evonik Nutrition & Care GmbH, 38%-ig, (INCI: Cocamidopropyl Betaine) | 7,90% |
| Sodium Chloride | 1,00% |
| Perfume, preservative | q.s. |
| Citric Acid, 30%-ig | q.s. to pH 5.0 |

Formulierungsbeispiel 27) Shampoo, PEG-frei (Nicht erfindungsgemäß)

| | |
|---|---|
| TEXAPON® LS 35, BASF, 30%, (INCI: Sodium Lauryl Sulfate) | 28,00% |
| Water | ad 100,00% |

(fortgesetzt)

| | |
|---|---|
| UCARE Polymer JR-400, Dow Chemicals, (INCI: Polyquaternium-10) | 0,10% |
| Jaguar C-162, Solvay, (INCI: Hydroxypropyl Guar Hydroxypropyltrimonium Chloride) | 0,10% |
| ANTIL® CM 90, Evonik Nutrition & Care GmbH, (INCI: Cocamide MEA) | 0,50% |
| ANTIL® SPA 80, Evonik Nutrition & Care GmbH, (INCI: Isostearamide MIPA; Glyceryl Laurate) | 1,00% |
| Xanthan Gum | 0,50% |
| Mikroemulsion ME3 | 3,00% |
| Argan Oil, DSM Nutritional Products Ltd., (INCI: Argania Spinosa Oil) | 0,10% |
| Dehyton AB 30, BASF, 31%, (INCI: Coco-Betaine) | 8,00% |
| Prifrac 2920, Croda, (INCI: Lauric Acid) | 0,50% |
| TEGOSOFT® PC 41, Evonik Nutrition & Care GmbH, (INCI: Polyglyceryl-4 Caprate) | 1,00% |
| Glycerin | 1,00% |
| Uvinul MS 40, BASF, (INCI: Benzophenon-4) | 0,10% |
| Versene 100, The Dow Chemical Company, (INCI: Tetrasodium EDTA) | 0,1% |
| Sodium Chloride | 1,00% |
| Perfume, preservative | q.s. |
| Citric Acid, 30%-ig | q.s. to pH 5.0 |

Formulierungsbeispiel 28) Shampoo, Sulfat-frei

| | |
|---|---|
| Bioterge AS-40 AOS, Stepan, (INCI: Sodium C14-16 Olefin Sulfonate) | 30,00% |
| Water | ad 100,00% |
| Jaguar C-162, Solvay, (INCI: Hydroxypropyl Guar Hydroxypropyltrimonium Chloride) | 0,15% |
| ANTIL® 500 Pellets, Evonik Nutrition & Care GmbH, (INCI: PEG-200 Glyceryl Stearate) | 0,20% |
| ANTIL® SPA 80, Evonik Nutrition & Care GmbH, (INCI: Isostearamide MIPA; Glyceryl Laurate) | 0,50% |
| Mikroemulsion ME2 | 3,50% |
| Cropure Avocado, Croda, (INCI: Persea Gratissima (Avocado) Oil) | 0,10% |
| Dehyton AB 30, BASF, 31%, (INCI: Coco-Betaine) | 8,00% |
| TAGAT® CH 40, Evonik Nutrition & Care GmbH, (INCI: PEG-40 Hydrogenated Castor Oil) | 1,00% |
| Glycerin | 1,00% |
| Uvinul MS 40, BASF, (INCI: Benzophenon-4) | 0,10% |
| Sodium Chloride | 1,50% |
| Perfume, preservative | q.s. |
| Citric Acid, 30%-ig | q.s. to pH 5.5 |

Formulierungsbeispiel 29) Shampoo, Sulfat-frei (Nicht erfindungsgemäß)

| | |
|---|---|
| TEGO@ Betain F 50, Evonik Nutrition & Care GmbH, 38%-ig, (INCI: Cocamidopropyl Betaine) | 22,00% |

(fortgesetzt)

| | |
|---|---|
| Water | ad 100,00% |
| Jaguar C-162, Solvay, (INCI: Hydroxypropyl Guar Hydroxypropyltrimonium Chloride) | 0,10% |
| ANTIL® 500 Pellets, Evonik Nutrition & Care GmbH, (INCI: PEG-200 Glyceryl Stearate) | 0,30% |
| REWOPAL® PEG 6000 DS A, Evonik Nutrition & Care GmbH, (INCI: PEG-150 Distearate) | 0,50% |
| Mikroemulsion ME3 | 2,50% |
| Cropure Olive, Croda, (INCI: Olea Europaea (Olive) Fruit Oil) | 0,10% |
| Hostapon SG, Clariant, (INCI: Sodium Cocoyl Glycinate) | 10,00% |
| TEGO@ Solve 61, Evonik Nutrition & Care GmbH, (INCI: Polyglyceryl-6 Caprylate; Polyglyceryl-3 Cocoate; Polyglyceryl-4 Caprate; Polyglyceryl-6 Ricinoleate) | 1,00% |
| Glycerin | 0,50% |
| Sodium Chloride | 1,00% |
| Perfume, preservative | q.s. |
| Citric Acid, 30%-ig | q.s. to pH 4.8 |

Formulierungsbeispiel 30) Rinse-Off Conditioner

| | |
|---|---|
| Water | ad 100,00% |
| VARISOFT® BT 85, Evonik Nutrition & Care GmbH, (INCI: Behentrimonium Chloride) | 2,00% |
| Mikroemulsion ME2 | 3,50% |
| TEGO® Alkanol 1618, Evonik Nutrition & Care GmbH, (INCI: Cetearyl Alcohol) | 5,00% |
| Citric Acid, 30%-ig | q.s. to pH 4,5 |
| Preservative, Perfume | q.s. |

Formulierunsbeisiel 31) Rinse-Off Conditioner

| | |
|---|---|
| Water | ad 100,00% |
| VARISOFT® EQ 65, Evonik Nutrition & Care GmbH, | 2,00% |
| (INCI: Distearoylethyl Dimonium Chloride; Cetearyl Alcohol) | |
| VARISOFT® BT 85, Evonik Nutrition & Care GmbH, (INCI: Behentrimonium Chloride) | 1,00% |
| Mikroemulsion ME19 | 1,80% |
| TEGO® Alkanol 1618, Evonik Nutrition & Care GmbH, (INCI: Cetearyl Alcohol) | 5,00% |
| Citric Acid, 30%-ig | q.s. to pH 4,0 |
| Preservative, Perfume | q.s. |

Formulierungsbeispiel 32) Rinse-Off Conditioner

| | |
|---|---|
| Water | ad 100,00% |

(fortgesetzt)

| | |
|---|---|
| VARISOFT® EQ 65, Evonik Nutrition & Care GmbH, (INCI: Distearoylethyl Dimonium Chloride; Cetearyl Alcohol) | 2,00% |
| VARISOFT® BT 85, Evonik Nutrition & Care GmbH (INCI: Behentrimonium Chloride) | 2,00% |
| ABIL® Quat 3272, Evonik Nutrition & Care GmbH, (INCI: Quaternium-80) | 0,50% |
| Mikroemulsion ME18 | 4,50% |
| TEGINACID® C, Evonik Nutrition & Care GmbH, (INCI: Ceteareth-25) | 0,50% |
| TEGO® Alkanol 1618, Evonik Nutrition & Care GmbH, (INCI: Cetearyl Alcohol) | 5,00% |
| Citric Acid, 30%-ig | q.s. to pH 3,5 |
| Preservative, Perfume | q.s. |

Formulierungsbeispiel 33) Rinse-Off Conditioner

| | |
|---|---|
| TEGINACID® C, Evonik Nutrition & Care GmbH, (INCI: Ceteareth-25) | 0,50% |
| TEGO® Alkanol 16, Evonik Nutrition & Care GmbH, (INCI: Cetyl Alcohol) | 2,00% |
| TEGO® Amid S 18, Evonik Nutrition & Care GmbH, (INCI: Stearamidopropyl Dimethylamine) | 1,00% |
| Mikroemulsion ME2 | 3,50% |
| Propylene Glycol | 2,00% |
| Citric Acid Monohydrate | 0,30% |
| Water | ad 100,00% |
| Preservative, Perfume | q.s. |

Formulierungsbeispiel 34) Rinse-Off Conditioner

| | |
|---|---|
| TEGINACID® C, Evonik Nutrition & Care GmbH, (INCI: Ceteareth-25) | 0,50% |
| TEGO® Alkanol 1618, Evonik Nutrition & Care GmbH, (INCI: Cetearyl Alcohol) | 6,50% |
| Mikroemulsion ME19 | 3,50% |
| Water | ad 100,00% |
| TEGO® Cosmo C 100, Evonik Nutrition & Care GmbH, (INCI: Creatine) | 0,50% |
| Propylene Glycol | 2,00% |
| Citric Acid, 30%-ig | q.s. to pH 4,0 |
| Preservative, Perfume | q.s. |

Formulierungsbeispiel 35) Rinse-Off Conditioner

| | |
|---|---|
| Water | ad 100,00% |
| TEGIN® M Pellets, Evonik Nutrition & Care GmbH, (INCI: Glyceryl Stearate) | 1,00% |
| Oleyl Alcohol | 0,70% |

(fortgesetzt)

| | |
|---|---|
| Glycerin | 1,50% |
| Mikroemulsion ME2 | 2,50% |
| TEGO® Alkanol 1618, Evonik Nutrition & Care GmbH, (INCI: Cetearyl Alcohol) | 4,50% |
| Citric Acid, 30%-ig | q.s. to pH 4,0 |
| Preservative, Perfume | q.s. |

Formulierungsbeispiel 36) Rinse-Off Conditioner

| | |
|---|---|
| Water | ad 100,00% |
| TEGIN® M Pellets, Evonik Nutrition & Care GmbH, (INCI: Glyceryl Stearate) | 1,00% |
| Oleyl Alcohol | 0,50% |
| Glycerin | 2,00% |
| Pure-Gel, Grain Processing Corporation, (INCI: Sodium Hydroxypropyl Starch Phosphate) | 1,50% |
| Mikroemulsion ME2 | 2,80% |
| TEGO® Alkanol 1618, Evonik Nutrition & Care GmbH, (INCI: Cetearyl Alcohol) | 6,00% |
| Citric Acid, 30%-ig | q.s. to pH 4,0 |
| Preservative, Perfume | q.s. |

Formulierungsbeispiel 37) Rinse-Off Conditioner (Nicht erfindungsgemäß)

| | |
|---|---|
| Water | ad 100,00% |
| TEGINACID® C, Evonik Nutrition & Care GmbH, (INCI: Ceteareth-25) | 1,00% |
| SF 1708, Momentive, (INCI: Amodimethicone) | 1,00% |
| DC 1503 Fluid, Dow Corning, (INCI: Dimethicone, Dimethiconol) | 0,50% |
| Mikroemulsion ME3 | 3,80% |
| TEGO® Alkanol 1618, Evonik Nutrition & Care GmbH, (INCI: Cetearyl Alcohol) | 6,00% |
| Citric Acid, 30%-ig | q.s. to pH 4,0 |
| Preservative, Perfume | q.s. |

Formulierungsbeispiel 38) Rinse-Off Conditioner

| | |
|---|---|
| Water | ad 100,00% |
| TEGIN® M Pellets, Evonik Nutrition & Care GmbH, (INCI: Glyceryl Stearate) | 1,00% |
| TEGO® Care PSC 3, Evonik Nutrition & Care GmbH, (INCI: Polyglyceryl-3 Dicitrate/Stearate) | 0,50% |
| Jaguar C-162, Solvay, (INCI: Hydroxypropyl Guar Hydroxypropyltrimonium Chloride) | 0,10% |
| Mikroemulsion ME2 | 5,00% |
| TEGO® Alkanol 1618, Evonik Nutrition & Care GmbH, (INCI: Cetearyl Alcohol) | 6,00% |

(fortgesetzt)

| | |
|---|---|
| Citric Acid, 30%-ig | q.s. to pH 4,5 |
| Preservative, Perfume | q.s. |

Formulierungsbeispiel 39) Rinse-Off Conditioner

| | |
|---|---|
| Water | ad 100,00% |
| TEGIN® M Pellets, Evonik Nutrition & Care GmbH, (INCI: Glyceryl Stearate) | 1,00% |
| Oleyl Alcohol | 0,30% |
| Lauryl Alcohol | 0,30% |
| TEGO® Care PSC 3, Evonik Nutrition & Care GmbH, (INCI: Polyglyceryl-3 Dicitrate/Stearate) | 0,80% |
| Glycerin | 0,50% |
| Mikroemulsion ME2 | 5,50% |
| TEGO® Alkanol 1618, Evonik Nutrition & Care GmbH, | 6,00% |
| (INCI: Cetearyl Alcohol) | |
| Citric Acid, 30%-ig | q.s. to pH 4,5 |
| Preservative, Perfume | q.s. |

Formulierungsbeispiel 40) Rinse-Off Conditioner

| | |
|---|---|
| Water | ad 100,00% |
| CORN PO4 PH "B", Agrana Starch, (INCI: Distarch Phosphate) | 4,00% |
| Mikroemulsion ME2 | 2,50% |
| TEGO® Alkanol 16, Evonik Nutrition & Care GmbH, (INCI: Cetyl Alcohol) | 1,00% |
| TEGO® Alkanol 18, Evonik Nutrition & Care GmbH, (INCI: Stearyl Alcohol) | 1,70% |
| Citric Acid, 30%-ig | q.s. to pH 4,3 |
| Preservative, Perfume | q.s. |

Formulierungsbeispiel 41) Leave-In Conditioner Spray

| | |
|---|---|
| Lactic Acid, 80% | 0,40% |
| Water | ad 100,00% |
| TEGO® Amid S 18, Evonik Nutrition & Care GmbH, (INCI: Stearamidopropyl Dimethylamine) | 1,00% |
| TEGIN® G 1100 Pellets, Evonik Nutrition & Care GmbH, (INCI: Glycol Distearate) | 0,60% |
| TEGO® Care PS, Evonik Nutrition & Care GmbH, (INCI: Methyl Glucose Sesquistearate) | 1,20% |
| Glycerin | 1,50% |
| Mikroemulsion ME2 | 3,00% |
| Preservative, Perfume | q.s. |

Formulierungsbeispiel 42) Leave-In Conditioner Spray

| | |
|---|---|
| TAGAT® CH-40, Evonik Nutrition & Care GmbH, (INCI: PEG-40 Hydrogenated Castor Oil) | 2,00% |
| Ceramide VI, Evonik Nutrition & Care GmbH, (INCI: Ceramide 6 II) | 0,10% |
| Perfume | 0,20% |
| Glycerin | 1,00% |
| Water | ad 100,00% |
| Mikroemulsion ME2 | 5,00% |
| LACTIL®, Evonik Nutrition & Care GmbH, | 2,00% |
| (INCI: Sodium Lactate; Sodium PCA; Glycine; Fructose; Urea; Niacinamide; Inositol; Sodium benzoate; Lactic Acid) | |
| TEGO® Betain F 50, Evonik Nutrition & Care GmbH, 38% (INCI: Cocamidopropyl Betaine) | 2,30% |
| Citric Acid, 10%-ig | 2,00% |

Formulierungsbeispiel 43) Leave-In Conditioner Foam (Nicht erfindungsgemäß)

| | |
|---|---|
| Mikroemulsion ME3 | 3,50% |
| TEGOSOFT® PC 41, Evonik Nutrition & Care GmbH, (INCI: Polyglyceryl-4 Caprate) | 3,00% |
| Perfume | 0,20% |
| TEGO® Betain 810, Evonik Nutrition & Care GmbH, (INCI: Capryl/Capramidopropyl Betaine) | 2,00% |
| Water | ad 100,00% |
| TEGO® Cosmo C 100, Evonik Nutrition & Care GmbH, (INCI: Creatine) | 0,50% |
| TEGOCEL® HPM 50, Evonik Nutrition & Care GmbH, (INCI: Hydroxypropyl Methylcellulose) | 0,30% |
| Citric Acid, 30%-ig | 0,10% |
| Preservative | q.s. |

Formulierungsbeispiel 44) Strong Hold Styling Gel

| | |
|---|---|
| TEGO® Carbomer 141, Evonik Nutrition & Care GmbH, (INCI: Carbomer) | 1,20% |
| Water | ad 100,00% |
| NaOH, 25%-ig | 2,60% |
| PVP/VA W-735, ISP, (INCI: PVP/VA Copolymer) | 16,00% |
| Mikroemulsion ME2 | 4,80% |
| Alcohol Denat. | 10,00% |
| TAGAT® O 2 V, Evonik Nutrition & Care GmbH, (INCI: PEG-20 Glyceryl Oleate) | 2,00% |
| Perfume | 0,30% |
| ABIL® B 88183 PH, Evonik Nutrition & Care GmbH, (INCI: PEG/PPG-20/6 Dimethicone) | 0,30% |
| Preservative | q.s. |

Formulierungsbeispiel 45) Spravable Hairmilk PEG-free

| | | |
|---|---|---|
| A | Water | ad 100,00% |
| | Lactic Acid, 80%-ig | 0,40% |
| B | Glycerin | 1,20% |
| | TEGIN® G 1100 Pellets, Evonik Nutrition & Care GmbH, (INCI: Glycol Distearate) | 0,50% |
| | TEGO® Care PS, Evonik Nutrition & Care GmbH, (INCI: Methyl Glucose Sesquistearate) | 1,20% |
| | Mikroemulsion ME18 | 2,50% |
| | Perfume, preservative | q.s. |

Formulierungsbeispiel 46) Haarfärbemittel (Nicht erfindungsgemäß)

| | |
|---|---|
| Water | ad 100,00% |
| TEGO® Alkanol 1618, Evonik Nutrition & Care GmbH, (INCI: Cetearyl Alcohol) | 10,00% |
| Eutanol® G, BASF, (INCI: Octyldodecanol) | 3,50% |
| REWOMID® C 212, Evonik Nutrition & Care GmbH, (INCI: Cocamide MEA) | 1,70% |
| Super Hartolan® B, Croda, (INCI: Lanolin Alcohol) | 3,00% |
| Avocadoöl, Henry Lamotte, (INCI: Persea Gratissima Oil) | 1,50% |
| Pristerene® 4960, Uniquema, (INCI: Stearic Acid) | 6,00% |
| EDTA BD, BASF, (INCI: Disodium EDTA) | 0,10% |
| Texapon® K12G, BASF, (INCI: Sodium Lauryl Sulfate) | 0,60% |
| Propylenglycol | 5,00% |
| Timica Silver Sparkle, BASF, (INCI: MICA; Titanium Dioxide) | 1,00% |
| Ammoniaklösung, 25%ig | 6,00% |
| 2,5-Diaminotoluolsulfat, (INCI: Toluene-2,5-Diamine) | 1,40% |
| Rodol® RS, Jos. H. Lowenstein & Sons, (INCI: Resorcinol) | 0,30% |
| HC Blue A42, (INCI: 2,4-Diaminophenoxyethanol di HCl) | 0,10% |
| Natriumsulfit | 0,50% |
| Perfume | 0,20% |
| Mikroemulsion ME3 | 1,00% |

Formulierungsbeispiel 47) Haarfärbemittel

| | |
|---|---|
| Water | ad 100,00% |
| TEGO® Alkanol 1618, Evonik Nutrition & Care GmbH, (INCI: Cetearyl Alcohol) | 12,00% |
| Super Hartolan® B, Croda, (INCI: Lanolin Alcohol) | 2,50% |
| Meadowfoam® Seed Oil, Fanning, (INCI: Limnanthes Alba) | 1,00% |
| Pristerene® 4960, Uniquema, (INCI: Stearic Acid) | 5,50% |

(fortgesetzt)

| | |
|---|---|
| EDTA BD, BASF, (INCI: Disodium EDTA) | 0,30% |
| Glycerin | 3,00% |
| Texapon® N 70, BASF, (INCI: Sodium Laureth Sulfate) | 2,00% |
| Monoethanolamin | 4,00% |
| 2,5-Diaminotoluolsulfat, (INCI: Toluene-2,5-Diamine) | 0,90% |
| Rodol® RS, Jos. H. Lowenstein & Sons, (INCI: Resorcinol) | 0,20% |
| Jarocol® 4A3MP, Vivimed Labs, (INCI: 4-Amino-M-Cresol) | 0,60% |
| Rodol® PAOC, Jos. H. Lowenstein & Sons, (INCI: 4-Amino-2-Hydroxytoluene) | 0,50% |
| Uantox® EBATE, Universal Preserv-A-Chem, (INCI: Erythorbic Acid) | 0,50% |
| Perfume | 0,20% |
| Mikroemulsion ME2 | 1,00% |

Formulierungsbeispiel 48) Haarfärbemittel

| | |
|---|---|
| Water | ad 100,00% |
| TEGO® Alkanol 1618, Evonik Nutrition & Care GmbH, (INCI: Cetearyl Alcohol) | 10,00% |
| Eutanol® G, BASF, (INCI: Octyldodecanol) | 1,00% |
| REWOMID® C 212, Evonik Nutrition & Care GmbH, (INCI: Cocamide MEA) | 2,00% |
| TEGIN® VS, Evonik Nutrition & Care GmbH, (INCI: Glyceryl Stearate SE) | 5,00% |
| Fitoderm®, Hispano Quimica S. A., (INCI: Squalane) | 1,00% |
| Coenzyme Q 10 | 0,10% |
| EDTA BD, BASF, (INCI: Disodium EDTA) | 0,30% |
| Texapon® K12G, BASF, (INCI: Sodium Lauryl Sulfate) | 0,20% |
| Propylenglycol | 5,00% |
| Ammoniaklösung, 25%ig | 3,00% |
| Rodol® ERN, Jos. H. Lowenstein & Sons, (INCI: 1-Naphthol) | 0,30% |
| Imexine® OAG, Chimex, (INCI: 2-Methyl-5-Hydroxyethylaminophenol) | 1,00% |
| Colorex® WP5, Teluca, (INCI: 1-Hydroxyethyl 4,5-Diamino Pyrazole Sulfate) | 2,60% |
| Ascorbinsäure | 0,20% |
| Perfume | 0,30% |
| Mikroemulsion ME6 | 2,50% |

## Patentansprüche

**1.** Zusammensetzung enthaltend

A) mindestens eine nicht silikonhaltige quaternäre Ammoniumverbindung ausgewählt aus der Gruppe der Esterquats,
B) mindestens ein kosmetisches Öl umfassend keine Silikonöle,
C) mindestens ein nicht-ionisches Tensid,

D) mindestens ein nicht-wässriges Lösungsmittel,
E) Wasser,

**dadurch gekennzeichnet, dass** sie eine Mikroemulsion ist.

2. Zusammensetzung gemäß Anspruch1, **dadurch gekennzeichnet, dass** A) ausgewählt ist aus der Gruppe der flüssigen Esterquats.

3. Zusammensetzung gemäß Anspruch 1 oder 2, **dadurch gekennzeichnet, dass**

A) in einer Menge von 1 Gew.-% bis 40 Gew.-%, bevorzugt in einer Menge von 1,5 Gew.-% bis 30 Gew.-%, besonders bevorzugt in einer Menge von 5 Gew.-% bis 25 Gew.-%,
B) in einer Menge von 5 Gew.-% bis 60 Gew.-%, bevorzugt in einer Menge von 10 Gew.-% bis 50 Gew.-%, besonders bevorzugt in einer Menge von 15 Gew.-% bis 40 Gew.-%,
C) in einer Menge von 5 Gew.-% bis 50 Gew.-%, bevorzugt in einer Menge von 10 Gew.-% bis 40 Gew.-%, besonders bevorzugt in einer Menge von 15 Gew.-% bis 35 Gew.-%,
D) in einer Menge von 2 Gew.-% bis 50 Gew.-%, bevorzugt in einer Menge von 3 Gew.-% bis 40 Gew.-%, besonders bevorzugt in einer Menge von 5 Gew.-% bis 30 Gew.-%, und
E) in einer Menge von 1 Gew.-% bis 60 Gew.-%, bevorzugt in einer Menge von 3 Gew.-% bis 50 Gew.-%, besonders bevorzugt in einer Menge von 5 Gew.-% bis 45 Gew.-%, enthalten ist, wobei sich die Gew.-% auf die Gesamtzusammensetzung beziehen.

4. Zusammensetzung gemäß mindestens einem der vorherigen Ansprüche, **dadurch gekennzeichnet, dass** die Masse der Komponente B) größer als die Masse der Komponente A) in der Zusammensetzung ist.

5. Zusammensetzung gemäß mindestens einem der vorherigen Ansprüche, **dadurch gekennzeichnet, dass**

A) ausgewählt ist aus der Gruppe der flüssigen Esterquats bestehend aus quaternierten Fettsäurealkanolaminester-Salze, besonders bevorzugt aus den Gruppen der quaternierten Fettsäureethanolaminester-Salze und der quaternierten Fettsäureisopropanolaminester-Salze.

6. Zusammensetzung gemäß mindestens einem der vorherigen Ansprüche, **dadurch gekennzeichnet, dass**

A) ausgewählt ist aus der Gruppe der flüssigen Esterquats umfassend Verbindungen der allgemeinen Formel (I)

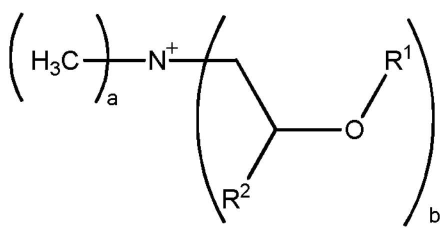

$X^-$ allgemeine Formel (I)

mit $R^1$ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit $R^2$ ein Alkylrest mit 1 bis 6 Kohlenstoffatomen, bevorzugt Methyl, Ethyl, Propyl, Isopropyl, besonders bevorzugt Methyl, und
mit X- ein Gegenion zu der positiven Ladung an der quartären Stickstoffgruppe,
wobei a = 1 bis 3 und b = 1 bis 3, bevorzugt a = 1,7 bis 2,3 und b = 1,7 bis 2,3 mit der Maßgabe, dass a + b =4.

7. Zusammensetzung gemäß mindestens einem der vorherigen Ansprüche, **dadurch gekennzeichnet, dass** Komponente C) ausgewählt ist aus solchen der allgemeinen Formel IV

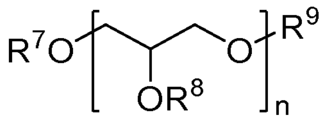

allgemeine Formel IV
mit

n = 2 bis 16, bevorzugt 3 - 14, besonders bevorzugt 4 - 11, und
$R^7$, $R^8$, $R^9$ = unabhängig voneinander gleich oder verschieden ausgewählt aus H und gegebenenfalls mindestens eine Hydroxylgruppe enthaltender, gesättigter oder ungesättigter, linearer oder verzweigter Acylrest mit 4 - 36 C-Atomen, insbesondere ausgewählt aus den Acylresten natürlicher Fettsäuren.

**8.** Zusammensetzung gemäß mindestens einem der vorherigen Ansprüche, **dadurch gekennzeichnet, dass** D) ausgewählt ist aus der Gruppe bestehend aus Hydrotrope und Polyole.

**9.** Zusammensetzung gemäß mindestens einem der vorherigen Ansprüche, **dadurch gekennzeichnet, dass** sie
F) amphoteres Tensid
enthält.

**10.** Verfahren zur Herstellung von Pflege- und Reinigungsformulierungen umfassend die Verfahrensschritte:

1) Bereitstellen einer Zusammensetzung gemäß mindestens einem der Ansprüche 1 bis 9,
2) Vermischen mit einer wasserhaltigen Phase.

**11.** Pflege- und Reinigungsformulierungen enthaltend eine Zusammensetzung gemäß mindestens einem der Ansprüche 1 bis 8 oder erhältlich nach dem Verfahren gemäß Anspruch 10.

## Claims

**1.** Composition comprising

A) at least one non-silicone-containing quaternary ammonium compound selected from the group of the ester quats,
B) at least one cosmetic oil comprising no silicone oils,
C) at least one nonionic surfactant,
D) at least one nonaqueous solvent,
E) water,

**characterized in that** it is a microemulsion.

**2.** Composition according to Claim 1, **characterized in that** A) is selected from the group of the liquid ester quats.

**3.** Composition according to Claim 1 or 2, **characterized in that** it contains

A) in an amount of 1 wt% to 40 wt%, preferably in an amount of 1.5 wt% to 30 wt%, more preferably in an amount of 5 wt% to 25 wt%,
B) in an amount of 5 wt% to 60 wt%, preferably in an amount of 10 wt% to 50 wt%, more preferably in an amount of 15 wt% to 40 wt%,
C) in an amount of 5 wt% to 50 wt%, preferably in an amount of 10 wt% to 40 wt%, more preferably in an amount of 15 wt% to 35 wt%,
D) in an amount of 2 wt% to 50 wt%, preferably in an amount of 3 wt% to 40 wt%, more preferably in an amount of 5 wt% to 30 wt%, and
E) in an amount of 1 wt% to 60 wt%, preferably in an amount of 3 wt% to 50 wt%, more preferably in an amount of 5 wt% to 45 wt%,

where the percentages by weight refer to the total composition.

**4.** Composition according to at least one of the preceding claims, **characterized in that** the mass of the component B) is greater than the mass of the component A) in the composition.

**5.** Composition according to at least one of the preceding claims, **characterized in that** A) is selected from the group of the liquid ester quats consisting of quaternized fatty acid alkanolamine ester salts, more preferably from the groups of the quaternized fatty acid ethanolamine ester salts and the quaternized fatty acid isopropanolamine ester salts.

**6.** Composition according to at least one of the preceding claims, **characterized in that**

A) is selected from the group of the liquid ester quats comprising

compounds of the general formula (I)

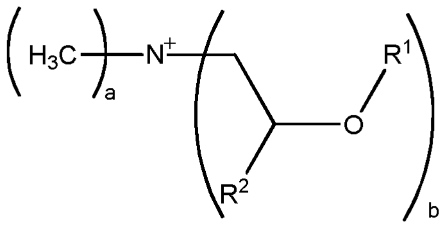

$X^-$ general formula (I)
where $R^1$ is an acyl radical of an at least monounsaturated fatty acid having a chain length of 18 to 24 carbon atoms or the acyl radical of isostearic acid or ricinoleic acid,
where $R^2$ is an alkyl radical having 1 to 6 carbon atoms, preferably methyl, ethyl, propyl or isopropyl, more preferably methyl, and
where $X^-$ is a counterion to the positive charge on the quaternary nitrogen group,
where a = 1 to 3 and b = 1 to 3, preferably a = 1.7 to 2.3 and b = 1.7 to 2.3,
with the proviso that a + b = 4.

**7.** Composition according to at least one of the preceding claims, **characterized in that** component C) is selected from those of the general formula IV

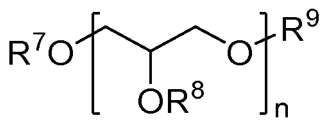

general formula IV
where

n = 2 to 16, preferably 3-14, more preferably 4-11, and
$R^7$, $R^8$, $R^9$ = are independently the same or different and are selected from H and saturated or unsaturated, linear or branched acyl radical having 4-36 carbon atoms and optionally containing at least one hydroxyl group, especially selected from the acyl radicals of natural fatty acids.

**8.** Composition according to at least one of the preceding claims, **characterized in that** D) is selected from the group consisting of hydrotropes and polyols.

**9.** Composition according to at least one of the preceding claims, **characterized in that** it comprises F) amphoteric surfactant.

**10.** Process for producing care and cleaning formulations, comprising the process steps of:

1) providing a composition according to at least one of Claims 1 to 9,
2) mixing with an aqueous phase.

**11.** Care and cleaning formulations containing a composition according to at least one of Claims 1 to 8, or obtainable by the process according to Claim 10.

## Revendications

**1.** Composition contenant

A) au moins un composé d'ammonium quaternaire ne contenant pas de silicone, choisi dans le groupe des esters d'ammonium quaternaire,
B) au moins une huile cosmétique ne comprenant pas d'huiles de silicone,
C) au moins un tensioactif non ionique,
D) au moins un solvant non aqueux,
E) de l'eau,

**caractérisée en ce qu'**elle est une microémulsion.

**2.** Composition selon la revendication 1, **caractérisée en ce que** A) est choisi dans le groupe des esters d'ammonium quaternaire liquides.

**3.** Composition selon la revendication 1 ou 2, **caractérisée en ce que**

A) est contenu en une quantité de 1 % en poids à 40 % en poids, de préférence en une quantité de 1,5 % en poids à 30 % en poids, de façon particulièrement préférée en une quantité de 5 % en poids à 25 % en poids,
B) est contenu en une quantité de 5 % en poids à 60 % en poids, de préférence en une quantité de 10 % en poids à 50 % en poids, de façon particulièrement préférée en une quantité de 15 % en poids à 40 % en poids,
C) est contenu en une quantité de 5 % en poids à 50 % en poids, de préférence en une quantité de 10 % en poids à 40 % en poids, de façon particulièrement préférée en une quantité de 15 % en poids à 35 % en poids,
D) est contenu en une quantité de 2 % en poids à 50 % en poids, de préférence en une quantité de 3 % en poids à 40 % en poids, de façon particulièrement préférée en une quantité de 5 % en poids à 30 % en poids, et
E) est contenu en une quantité de 1 % en poids à 60 % en poids, de préférence en une quantité de 3 % en poids à 50 % en poids, de façon particulièrement préférée en une quantité de 5 % en poids à 45 % en poids,

les % en poids se rapportant à la composition totale.

**4.** Composition selon au moins l'une quelconque des revendications précédentes, **caractérisée en ce que** la masse du composant B) est supérieure à la masse du composant A) dans la composition.

**5.** Composition selon au moins l'une quelconque des revendications précédentes, **caractérisée en ce que**

A) est choisi dans le groupe des esters d'ammonium quaternaire liquides constitué par les sels d'esters d'alcanolamines avec des acides gras, quaternisés, de façon particulièrement préférée dans les groupes des sels d'esters d'éthanolamine avec des acides gras, quaternisés, et des sels d'esters d'isopropanolamine avec des acides gras, quaternisés.

**6.** Composition selon au moins l'une quelconque des revendications précédentes, **caractérisée en ce que**

A) est choisi dans le groupe des esters d'ammonium quaternaire liquides comprenant les composés de formule générale (I)

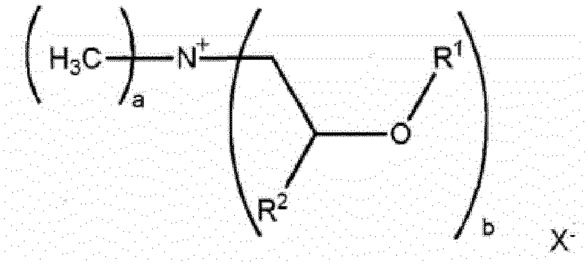

formule Générale (I)

où $R^1$ est un radical acyle d'un acide gras au moins une fois insaturé ayant une longueur de chaîne de 18 à 24 atomes de carbone ou le radical acyle de l'acide isostéarique ou de l'acide ricinoléique,
où $R^2$ est un radical alkyle ayant de 1 à 6 atomes de carbone, de préférence méthyle, éthyle, propyle, isopropyle, de façon particulièrement préférée méthyle, et
où $X^-$ est un ion opposé à la charge positive sur le groupe à azote quaternaire,
où a = 1 à 3 et b = 1 à 3, de préférence a = 1,7 à 2,3 et b = 1,7 à 2,3, étant entendu que a + b = 4.

**7.** Composition selon au moins l'une quelconque des revendications précédentes, **caractérisée en ce que** Le composant C) est choisi parmi ceux de formule générale IV

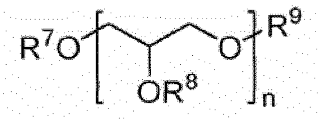

formule générale IV
où

n = 2 à 16, de préférence 3 - 14, de façon particulièrement préférée 4 - 11, et
$R^7$, $R^8$, $R^9$ = identiques ou différents, sont choisis indépendamment les uns des autres parmi H et un radical acyle linéaire ou ramifié, saturé ou insaturé, ayant de 4 à 36 atomes de carbone, contenant éventuellement au moins un groupe hydroxy, en particulier choisi parmi les radicaux acyle d'acides gras naturels.

**8.** Composition selon au moins l'une quelconque des revendications précédentes, **caractérisée en ce que** D) est choisi dans le groupe constitué par les hydrotropes et les polyols.

**9.** Composition selon au moins l'une quelconque des revendications précédentes, **caractérisée en ce qu'**elle contient F) un tensioactif amphotère.

**10.** Procédé pour la préparation de formulations de soins et de nettoyage, comprenant les étapes de processus :

1) fourniture d'une composition selon au moins l'une quelconque des revendications 1 à 9,
2) mélange avec une phase aqueuse.

**11.** Formulations de soins et de nettoyage contenant une composition selon au moins l'une quelconque des revendications 1 à 8 ou pouvant être obtenue conformément au procédé selon la revendication 10.

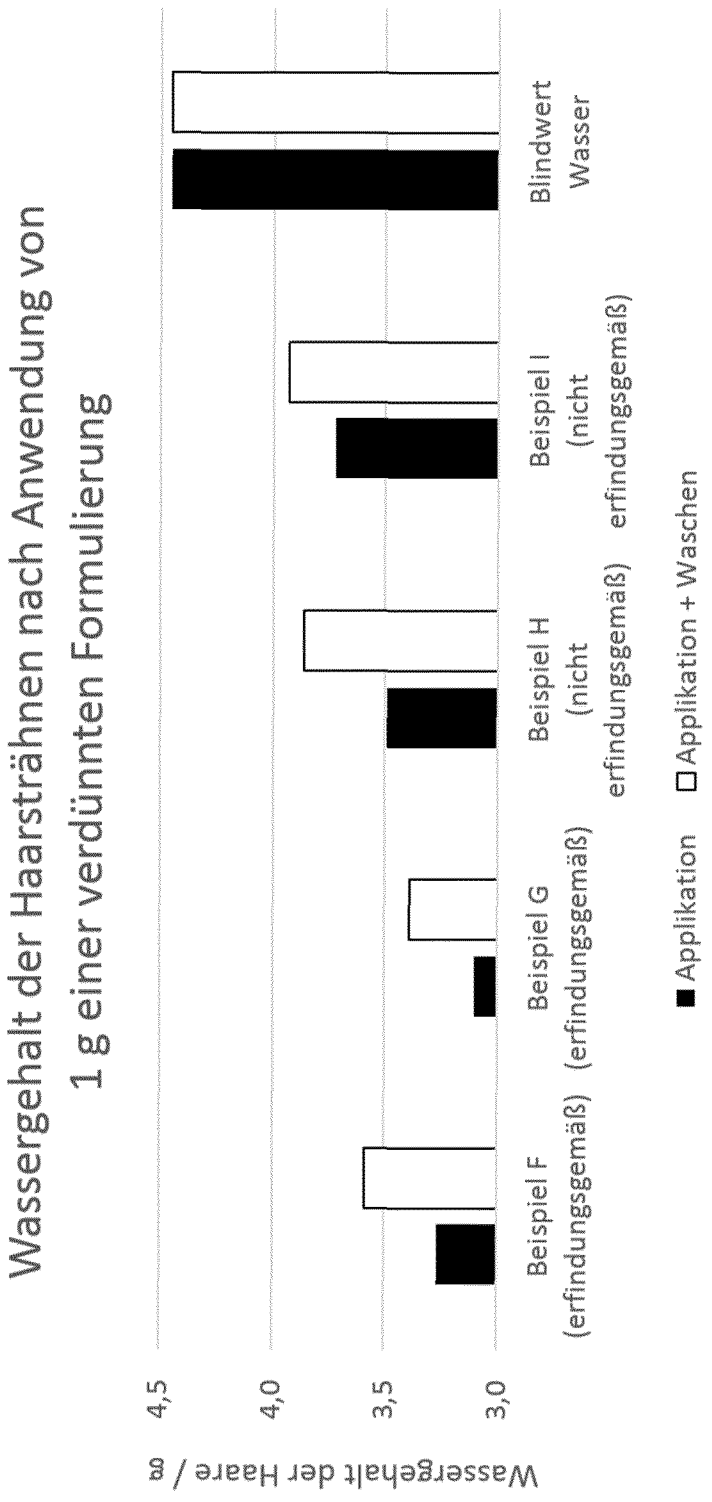
Wassergehalt der Haarsträhnen nach Anwendung von 1 g einer verdünnten Formulierung
Wassergehalt der Haare / g
4,5
4,0
3,5
3,0
Beispiel F (erfindungsgemäß)
Beispiel G (erfindungsgemäß)
Beispiel H (nicht erfindungsgemäß)
Beispiel I (nicht erfindungsgemäß)
Blindwert Wasser
Applikation
Applikation + Waschen

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008155075 A
- EP 1715833 A
- WO 2008155073 A
- DE 19755488
- US 2013012423 A
- DE 102011078382
- EP 2273966 A1
- DE 10327871
- FR 6935


### In der Beschreibung aufgeführte Nicht-Patentliteratur


- Neutrons, X-Rays and Light: Scattering Methods Applied to Soft Condensed Matter. **P. LINDNER ; TH. ZEMB.** Science & Technology. Elsevier, November 2002
- **O. GLATTER ; O. KRATKY.** Small-angle X-ray Scattering. Academic Press Inc
- **CASSEL et al.** Original synthesis of linear, branched and cyclic oligoglycerol standards. *J. Org. Chem.,* 2001, 875-896
- **BEISPIEL K. SCHRADER.** Grundlagen und Rezepturen der Kosmetika. *Handbücher,* 329-341